# EUROPEAN PATENT SPECIFICATION

(11) **EP 4 169 573 B1**
(45) Date of publication and mention of the grant of the patent: **26.11.2025**
(21) Application number: 21826580.9
(22) Date of filing: 10.06.2021
(51) Int. Cl.: A61N 5/06, C09K 11/80

(54) **LIGHT IRRADIATION TYPE COSMETIC APPARATUS AND ELECTRONIC DEVICE**
LICHTBESTRAHLUNGSKOSMETIKVORRICHTUNG UND ELEKTRONISCHE VORRICHTUNG
APPAREIL COSMÉTIQUE DE TYPE À RAYONNEMENT LUMINEUX ET DISPOSITIF ÉLECTRONIQUE

(30) Priority: 19.06.2020 JP 2020106043
(43) Date of publication of application: 26.04.2023
(73) Proprietor: Panasonic Intellectual Property Management Co., Ltd., Osaka-shi, Osaka 540-6207 (JP)
(72) Inventor: NITTA, Mitsuru, Osaka-shi, Osaka 540-6207 (JP); OSHIO, Shozo, Osaka-shi, Osaka 540-6207 (JP); FUJIWARA, Chigusa, Osaka-shi, Osaka 540-6207 (JP); SHIGITANI, Ryosuke, Osaka-shi, Osaka 540-6207 (JP)
(74) Representative: Müller-Boré & Partner Patentanwälte PartG mbB
(86) International application number: PCT/JP2021/022112
(87) International publication number: WO 2021/256369

(56) References cited:
- WO-A1-2014/084379
- WO-A1-2020/137731
- WO-A1-2020/137731
- WO-A1-2020/162243
- WO-A1-2020/217671
- CN-A- 110 518 004
- JP-A- 2007 087 834
- JP-A- 2015 528 338
- US-A1- 2014 248 678
- QIYUE SHAO ET AL: "Broadband near-infrared light source derived from Cr 3+ -doped phosphors and a blue LED chip", OPTICS LETTERS, vol. 43, no. 21, 1 November 2018 (2018-11-01), US, pages 5251, XP055641220, ISSN: 0146-9592, DOI: 10.1364/OL.43.005251
- XU TAO ET AL: "Y3Al5O12:Ce3+single crystal and red-emitting Y3Al5O12:Cr3+single crystal for high power W-LEDs", OPTICAL MATERIALS, vol. 91, 16 March 2019 (2019-03-16), pages 30 - 34, XP085697202, ISSN: 0925-3467, DOI: 10.1016/J.OPTMAT.2019.03.010

## Description

### TECHNICAL FIELD

The present disclosure relates to a light irradiation type cosmetic apparatus and an electronic device.

### BACKGROUND ART

A light irradiation type cosmetic apparatus has been recently known for performing photocosmetic treatment, such as taking care of unwanted hair, removal of spots and freckles, or the like, through irradiation of skin with near-infrared light. For example, irradiation of hair cells with near-infrared light of relatively high intensity is known to be effective for hair removal because proteins associated with melanin pigments are denatured through heat coagulation, thereby reducing the hair regenerative function. It is known that near-infrared light of relatively high intensity is also effective in removing spots and freckles. Thus, when near-infrared light is used at high-output within a range that does not have a harmful influence on the human body, the effect of photocosmetic treatment is likely to be high. LEDs, xenon lamps, and the like are known as a light source of high-output near-infrared light.

Note that when a xenon lamp is used, the output light of the xenon lamp includes light in a wavelength range shorter than that of near-infrared light. Thus, a light irradiation type cosmetic apparatus using a xenon lamp light source is not energy efficient because light in the wavelength range of 550 nm or less is usually cut off by a filter. Then, as a light source or a light emitting device for a light irradiation type cosmetic apparatus, one that can emit a large amount of a light component of near-infrared light is desired. Here, a light emitting device including a phosphor that emits near-infrared light is being considered. Note that the light output of a phosphor generally tends to be saturated when the phosphor is irradiated with high-output light. Thus, a light emitting device capable of outputting high-output near-infrared light is desired. Various conventional light emitting devices have been considered as a light emitting device capable of outputting high-output near-infrared light.

For example, a configuration (P) light emitting device using a Cr³⁺-activated phosphor is known. Also, a configuration (Q) light emitting device including an LED chip that emits incoherent light and including a near-infrared phosphor is known. Furthermore, a configuration (R) light emitting device including a light source that emits coherent laser light, such as a laser diode, and including a phosphor (hereinafter called a "red phosphor") that emits a red fluorescent component is known.

Patent Literature 1 discloses a light emitting device using a YAG-based phosphor co-activated with Cr³⁺ and Ce³⁺, as a light emitting device satisfying the configurations (P) and (Q). Examples of the above-described YAG-based phosphor used include Y₃Al₅O₁₂:Cr³⁺, Ce³⁺, Lu₃Al₅O₁₂:Cr³⁺, Ce³⁺, Y₃(Al, Ga)₅O₁₂:Cr³⁺, Ce³⁺, and (Y, Gd)₃Al₅O₁₂:Cr³⁺, Ce³⁺.

Patent Literature 2 discloses an illumination light source for plant growth using a phosphor having a fluorescence peak in a wavelength range of 700 to 760 nm corresponding to a light absorption spectrum of pigment proteins (phytochromes) possessed by plants, as a light emitting device satisfying the configurations (P) and (Q). Specifically, Patent Literature 2 discloses a plant-growth illumination light source that packages a blue LED and a Gd₃Ga₅O₁₂:Cr³⁺ phosphor having a fluorescence peak in the wavelength range of 700 to 760 nm. With this illumination light source, the wavelength range of 700 to 760 nm where the fluorescent peak of the phosphor exists corresponds to the light absorption spectrum of the pigment protein (phytochromes), and thus it is possible to control the growth and differentiation of plants. Patent Literature 6 discloses an infrared light emitting device that emits light in a wide band in a wavelength range where the light reception sensitivity of an Si photodiode detector is high.

Patent Literature 3 discloses a medical inspection device that outputs a reflected image and a transmission image of a near-infrared light component emitted on living tissue, as a light emitting device satisfying the configuration (Q). This medical inspection device uses, as a near-infrared light component, a fluorescent component emitted by a phosphor containing the rare earths Nd and Yb as activators.

Patent Literature 4 discloses a light device in which various lasers are applied, including a laser diode, and including a red phosphor activated with Ce³⁺, as a light emitting device satisfying the configuration (R).

Note that the light emitting devices described in Patent Literatures 1 to 3 and 6 that do not satisfy the configuration (R) are for the purpose of providing lighting devices for growing plants, and the like, simply to obtain output light containing a near-infrared light component suitable for growing plants, and the like. That is, the light emitting devices described in Patent Literatures 1 to 3 and 6 do not solve the issue in which the light output of a phosphor is saturated, which is inherent in a light emitting device using high-output light such as laser light. Thus, the light emitting devices described in Patent Literatures 1 to 3 and 6 do not limit, in an extreme manner, the shape and the like of the fluorescence spectrum emitted by the Cr³⁺-activated phosphor in order to solve the issue in which the light output of the phosphor is saturated.

As a light emitting device using a near-infrared phosphor, a lighting device mainly for plant growth is known. However, this light emitting device is simply for obtaining output light containing a near-infrared light component suitable for growing plants, and does not solve the issue in which the light output of a phosphor is saturated when the phosphor is subjected to high-density photoexcitation.

As a light emitting device using high-output light such as laser light, a light emitting device that obtains visible output light using a phosphor activated mainly with a rare earth ion (Ce³⁺ or Eu²⁺) is known. However, this light emitting device does not provide near-infrared high-output light based on a Cr³⁺ electron energy transition.

As described above, a light emitting device where a phosphor is excited by high-output light such as laser light has had the issue until now in which the fluorescence output of the phosphor is saturated. To suppress the saturation of the fluorescence output, it has been considered essential to use a phosphor having a short afterglow (10 µs or less), which exhibits fluorescence based on a parity-allowed transition, such as Ce³⁺ or Eu²⁺, as described in Patent Literatures 4 and 5. In particular, the use of a Ce^{3 +}-activated phosphor exhibiting an ultrashort afterglow (10 to 100 ns) was considered to be preferable.

A light source based on a Cr³⁺ electron energy transition is also known from CN110518004A.

### CITATION LIST

### PATENT LITERATURE

Patent Literature 1: Japanese Unexamined Patent Application Publication No. 2016-121226
Patent Literature 2: WO 2010/053341
Patent Literature 3: Japanese Patent No. 5812461
Patent Literature 4: Japanese Patent No. 6206696
Patent Literature 5: WO 2016/092743
Patent Literature 6: WO 2018/207703

### SUMMARY OF THE INVENTION

However, in a light emitting device in which a phosphor is excited by laser light, the following issues arise when the near-infrared light component necessary in a light irradiation type cosmetic apparatus is obtained by using a Ce³⁺-activated phosphor or an Eu²⁺-activated phosphor. That is, it is difficult to develop a phosphor because the selection of materials used for the phosphor is narrow and the temperature quenching becomes large, and thus a light emitting device that emits a near-infrared light component cannot be obtained.

The present disclosure has been made in consideration of these issues. The present disclosure is achieved by finding that when a phosphor having Cr³⁺ as an activator and emitting long-afterglow (10 µs or more) fluorescence based on a parity-forbidden transition is used, the saturation of fluorescence output is less likely to occur even under photoexcitation emitted from a solid-state light source having a high-density rated output of 1 W or more, which is contrary to conventional common technical knowledge.

The above-described finding is surprising because it differs greatly from the conventional common technical knowledge that the use of a phosphor having a short afterglow (less than 10 µs) is essential to suppress the saturation of fluorescence output.

An object of the present disclosure is to provide a light irradiation type cosmetic apparatus that emits high-output light having a high proportion of a near-infrared fluorescent component under excitation with high-output light, and an electronic device using the light irradiation type cosmetic apparatus.

In response to the above issues, a light irradiation type cosmetic apparatus according to an aspect of the present disclosure includes a light emitting device including a light source that emits primary light, and a first phosphor that absorbs the primary light and converts the primary light into first wavelength-converted light having a wavelength longer than that of the primary light, wherein the light source is a solid-state light source having a rated output of 1 W or more, the primary light is light emitted from the solid-state light source, the first wavelength-converted light contains fluorescence based on an electron energy transition of Cr³⁺, and the first wavelength-converted light has a fluorescence spectrum having a fluorescence intensity maximum value in a wavelength range exceeding 710 nm.

An electronic device according to another aspect of the present disclosure includes the light irradiation type cosmetic apparatus.

The invention is defined in the appended claims.

### BRIEF DESCRIPTION OF DRAWINGS

[Fig. 1] Fig. 1 is a plan view of an example of a light irradiation type cosmetic apparatus according to an embodiment.
[Fig. 2] Fig. 2 is a side view of the example of the light irradiation type cosmetic apparatus according to the embodiment.
[Fig. 3] Fig. 3 is a partial cross-sectional view including a cross-section along the A-A line of Fig. 2.
[Fig. 4] Fig. 4 is a schematic cross-sectional view of an example of a light emitting device (first light emitting device) included in the light irradiation type cosmetic apparatus.
[Fig. 5] Fig. 5 is a schematic cross-sectional view of a more specific example of the light emitting device (second light emitting device) included in the light irradiation type cosmetic apparatus.
[Fig. 6] Fig. 6 is a schematic cross-sectional view of a more specific example of the light emitting device (third light emitting device) included in the light irradiation type cosmetic apparatus.
[Fig. 7] Fig. 7 is a schematic cross-sectional view of a more specific example of the light emitting device (fourth light emitting device) included in the light irradiation type cosmetic apparatus.
[Fig. 8] Fig. 8 is a schematic cross-sectional view of a more specific example of the light emitting device (fifth light emitting device) included in the light irradiation type cosmetic apparatus.
[Fig. 9] Fig. 9 illustrates electron energy levels of Cr³⁺.
[Fig. 10] Fig. 10 illustrates a relationship between wavelengths of light and absorption ratios of light in various substances.
[Fig. 11] Fig. 11 is a graph illustrating a relationship between wavelengths and PL intensities.
[Fig. 12] Fig. 12 is a graph illustrating a relationship between damping rates and PL intensities.
[Fig. 13] Fig. 13 is a graph illustrating a relationship between excitation light power densities and PL intensities.

### DESCRIPTION OF EMBODIMENTS

A light irradiation type cosmetic apparatus according to the present embodiment is described below with reference to the drawings. Note that dimensional ratios in the drawings are exaggerated for convenience of explanation and may differ from the actual ratios.

### [Light irradiation type cosmetic apparatus]

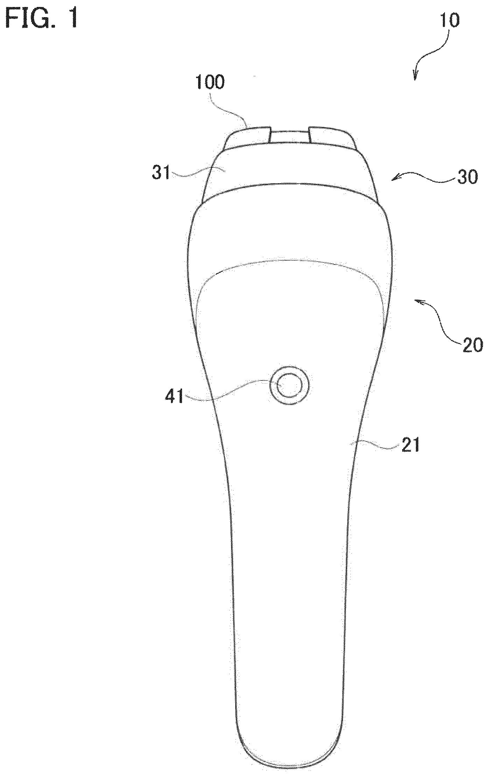

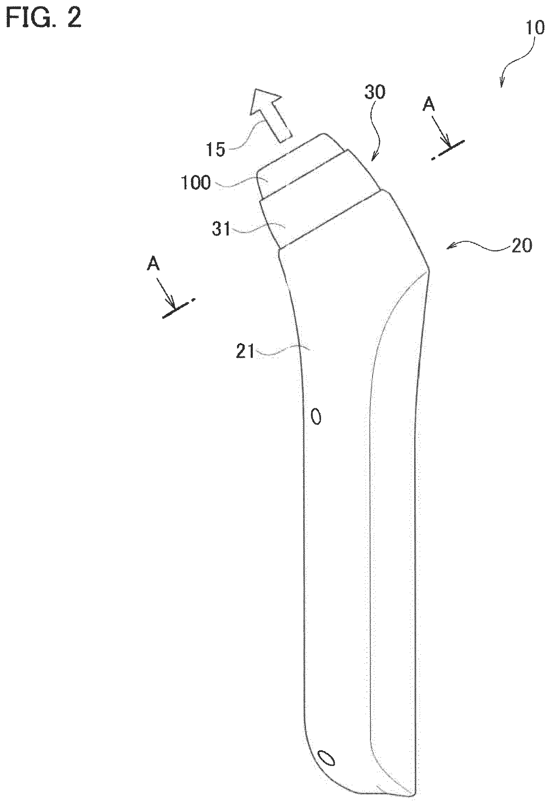

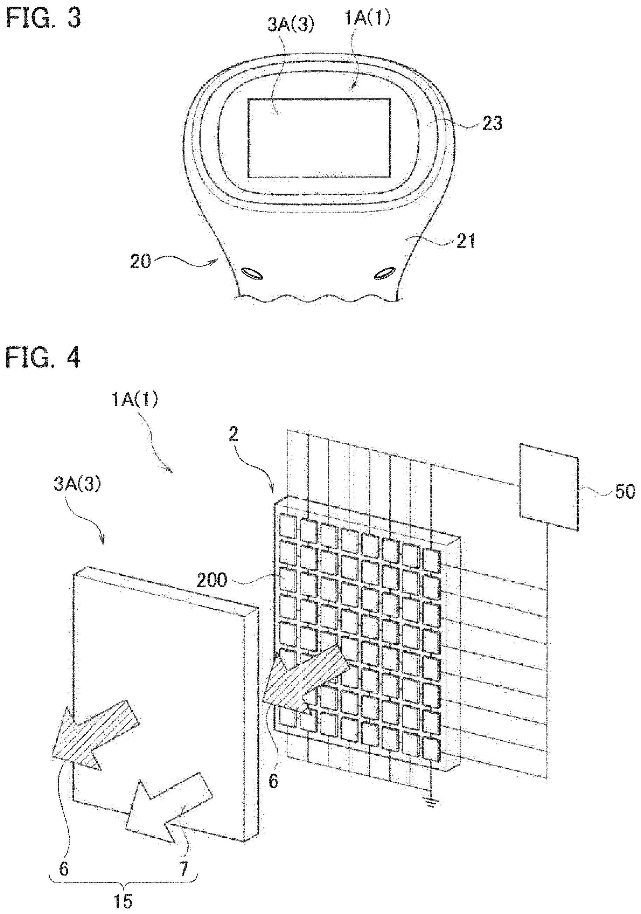

Fig. 1 is a plan view of an example of a light irradiation type cosmetic apparatus according to an embodiment. Fig. 2 is a side view of the example of the light irradiation type cosmetic apparatus according to the embodiment. Fig. 3 is a partial cross-sectional view including a cross-section along the A-A line of Fig. 2. Specifically, the cross section in Fig. 3 illustrates a cross section along a head-side end part 23 of a housing 21 of a body part 20, in which the body part 20 joins a head part 30. Fig. 4 is a schematic cross-sectional view of an example of the light emitting device (first light emitting device) included in the light irradiation type cosmetic apparatus. Note that more specific examples of the light emitting device (second to fifth light emitting devices) included in the light irradiation type cosmetic apparatus are described later.

As illustrated in Figs. 1 and 2, a light irradiation type cosmetic apparatus 10 according to the embodiment includes the body part 20 and a head part 30. The light irradiation type cosmetic apparatus 10 is capable of irradiating a target part of a person with output light 15 generated at the body part 20, through an irradiation port 110 provided in the head part 30.

As illustrated in Figs. 1 to 3, the body part 20 includes a hollow housing 21 having the head-side end part 23, which is an end part joined to the head part 30, and a light emitting device 1A (1) housed in the housing 21. The body part 20 emits the output light 15 emitted from the light emitting device 1A toward the head-side end part 23. The body part 20 includes a power button 41 to activate the light irradiation type cosmetic apparatus 10 or the like.

### (Light emitting device (first light emitting device))

As illustrated in Fig. 4, the light emitting device (first light emitting device) 1A includes the light source 2 that emits the primary light 6, and a wavelength converter 3A (3) including a first phosphor that absorbs the primary light 6 and converts it into first wavelength-converted light 7 having a wavelength longer than that of the primary light 6. Note that it is sufficient for the wavelength converter 3A (3) to include at least the first phosphor as a phosphor, and the wavelength converter 3A (3) may include a phosphor other than the first phosphor as necessary.

The light source 2 includes multiple solid-state light emitting elements 200. A driving power source 50 is connected to each of the solid-state light emitting elements 200 constituting the light source 2 to supply power to cause the solid-state light emitting elements 200 to emit light. When power is supplied from the driving power source 50 to the solid-state light emitting elements 200 by pressing the power button 41 or the like, the solid-state light emitting elements 200 of the light source 2 emit the primary light 6.

In the light emitting device 1A, the light source 2 and the wavelength converter 3A are arranged in such a manner that the primary light 6 emitted from the solid-state light emitting elements 200 of the light source 2 is emitted on the wavelength converter 3A including the first phosphor 4. The first phosphor 4 included in the wavelength converter 3A absorbs the primary light 6 emitted by the light source 2, converts it into the first wavelength-converted light 7 having a wavelength longer than that of the primary light 6, and emits it through the wavelength converter 3A. Note that part of the primary light 6 emitted from the light source 2 to the wavelength converter 3A usually penetrates or is reflected by the wavelength converter 3A. Thus, when the primary light 6 is emitted from the light source 2 to the wavelength converter 3A, the output light 15 including the primary light 6 and the first wavelength-converted light 7 is emitted from one surface of the wavelength converter 3A opposite to the other surface thereof facing the light source 2, for example.

In the light emitting device 1A illustrated in Fig. 3, the wavelength converter 3A is arranged on the body part 20 at a side thereof closer to the head part 30, and the light source 2, which is not illustrated, is arranged on the back side of the wavelength converter 3A. In the light emitting device 1A, the primary light 6 is emitted to the wavelength converter 3A from the light source 2 arranged on the back side of the wavelength converter 3A, and the output light 15 illustrated in Fig. 4 is emitted from the wavelength converter 3A toward the irradiation port 110 on the front side in the drawing of Fig. 3.

The head part 30 has a tubular head part body 31 and the irradiation port 110 arranged at the end of the head part body 31. The head part 30 is attached to the head-side end part 23 of the body part 20. The head part 30 outputs the output light 15 emitted from the light emitting device 1A of the body part 20 to the outside of the light irradiation type cosmetic apparatus 10 through the irradiation port 110. The head part 30 has a known structure in which the output light 15 introduced from the wavelength converter 3A of the body part 20 is emitted from the irradiation port 110.

When the output light 15 is emitted on human skin from the irradiation port 110 of the light irradiation type cosmetic apparatus 10, the output light 15 acts on melanin pigments to decrease a regenerative function of hair, and thus it is possible to obtain a temporary hair growth reduction effect and a hair growth suppression effect. It is also possible to obtain an effect of the removal of spots, freckles, and the like. Specifically, the first wavelength-converted light 7 included in the output light 15 is light that emits fluorescence based on an electron energy transition of Cr³⁺ and satisfying the following property (A) and contains a light component in a wavelength range suitable for easier absorption by melanin pigments than by blood and the like. Thus, when the output light 15 including the first wavelength-converted light 7 is emitted on human skin, a burn is less likely to occur, thermal efficiency is high, and destruction, coagulation, or the like occurs in melanin pigments and the like. When the melanin pigments and the like, which have been destroyed or coagulated, come to the surface through the skin's metabolism and are then peeled off and removed from the skin, it is expected that beautiful skin can be obtained through the suppression of unwanted hair growth and the removal of spots, freckles, and the like. The fact that the first wavelength-converted light 7 included in the output light 15 is light in a wavelength range suitable for easier absorption by melanin pigments than by blood and the like is described later.

In the light emitting device 1A of the light irradiation type cosmetic apparatus 10, when the primary light 6 emitted from the light source 2 enters the wavelength converter 3, a phosphor, such as the first phosphor 4, included in the wavelength converter 3 emits fluorescence. When receiving the primary light 6, the first phosphor 4 emits the first wavelength-converted light containing fluorescence based on the electron energy transition of Cr³⁺ and having a fluorescence intensity maximum value in a wavelength range exceeding 710 nm.

The light emitting device 1A of the light irradiation type cosmetic apparatus 10 emits the first wavelength-converted light that contains a greater amount of a broad spectral component having a fluorescence intensity maximum value in a wavelength range exceeding 710 nm, than a linear spectral component having a fluorescence intensity maximum value in a wavelength range of 680 to 710 nm. Thus, the light emitting device 1A is a light emitting device that contains a large amount of the near-infrared component.

Note that the above-described linear spectral component is a long-afterglow light component based on an electron energy transition (spin-forbidden transition) of²T₁ and ²E → ⁴A₂ of Cr³⁺. The above-described broad spectral component is a short-afterglow light component based on an electron energy transition (spin-allowed transition) of ⁴T₂ → ⁴A₂. The mechanism of fluorescence related to Cr³⁺ is described below.

The light source 2 and the wavelength converter 3A are described in detail below.

### <Light source>

The light source 2 is a solid-state light source having a rated output of 1 W or more and emits the primary light 6. That is, light emitted from a solid-state light source having a rated output of 1 W or more is used as the primary light 6. As the primary light 6 that is light emitted from a solid-state light source having a rated output of 1 W or more, warm color light having an intensity maximum value within a wavelength range of 600 nm or more and less than 710 nm is used, for example. As the warm color light, light having an intensity maximum value within a wavelength range of 600 nm or more and less than 680 nm is preferably used.

When the warm color light is used as the primary light 6, the primary light 6 is well absorbed by the first phosphor 4 activated with Cr³⁺ and is efficiently wavelength-converted to the first wavelength-converted light 7. Thus, it is possible for the light emitting device 1A and the light irradiation type cosmetic apparatus 10 including the light emitting device 1A to emit output light having a large proportion of a fluorescent component based on the electron energy transition of Cr³⁺.

It is preferable to use the warm color light as the primary light 6 because the Stokes shift between the primary light 6 in the first phosphor 4 and the first wavelength-converted light 7 becomes smaller and thus it becomes difficult for the light source 2 to generate heat.

It is preferable to use the warm color light as the primary light 6 because even when the primary light 6 penetrates the wavelength converter 3A and is emitted on human skin, it is less likely to have a harmful influence on human skin.

As the light source 2, for example, a solid-state light source, such as a warm-color laser element or a warm-color LED, emitting the above-described warm color light is used. When the light source 2 is a warm-color laser element or a warm-color LED, a phosphor in the wavelength converter 3 is excited with high efficiency, which enables the light emitting device 1A and the light irradiation type cosmetic apparatus 10 including the light emitting device 1A to emit high-output near-infrared light. Since the light source 2 is a solid-state light source, such as a laser element or an LED, it has excellent durability and a long life.

A red laser element or a red LED in the warm-color laser element or the warm color LED has a small energy difference from a near-infrared light component and a small energy loss due to wavelength conversion. Thus, it is preferable to use a red laser element or a warm color LED as the light source 2 for achieving the high efficiency of the light emitting device 1A and the light irradiation type cosmetic apparatus 10 including the light emitting device 1A.

For example, a surface emitting laser diode or an LED is used as the solid-state light emitting elements 200 constituting the light source 2. The light source 2 has a rated light output of 1 W or more, preferably 3 W or more. When the rated light output of the light source 2 is within the above-described ranges, the light source 2 emits the high-output primary light 6, and thus it is possible to achieve high output of the light emitting device 1A and the light irradiation type cosmetic apparatus 10 including the light emitting device 1A.

Note that there is no particular upper limit for the rated light output. It is possible for the light source 2 to achieve high output by increasing the number of solid-state light emitting elements 200. However, for practicality, the rated light output of the light source 2 is usually less than 10 kW, preferably less than 3 kW.

The power density of the primary light 6 emitted on the first phosphor 4 is usually 0.5 W/mm² or more, preferably more than 3 W/mm², more preferably more than 10 W/mm², even more preferably more than 30 W/mm². When the power density of the primary light 6 is within the above-described ranges, the first phosphor 4 is excited by high density light, and thus it becomes possible for the light emitting device 1A and the light irradiation type cosmetic apparatus 10 including the light emitting device 1A to emit a high-output fluorescent component.

### <Wavelength converter>

The wavelength converter 3A includes the first phosphor 4 and a sealant 5. In the wavelength converter 3A, the first phosphor 4 is usually included in the sealant 5.

### [First phosphor]

The first phosphor 4 is a phosphor that absorbs the primary light 6 and converts it into the first wavelength-converted light 7 having a wavelength longer than that of the primary light 6. The first phosphor 4 absorbs the primary light 6 and emits the first wavelength-converted light 7 containing fluorescence based on the electron energy transition of Cr³⁺. That is, the first wavelength-converted light 7 contains fluorescence based on the electron energy transition of Cr³⁺. Here, the fluorescence based on the electron energy transition of Cr³⁺ means fluorescence based on the electron energy transition (spin-allowed transition) of ⁴T₂ → ⁴A₂.

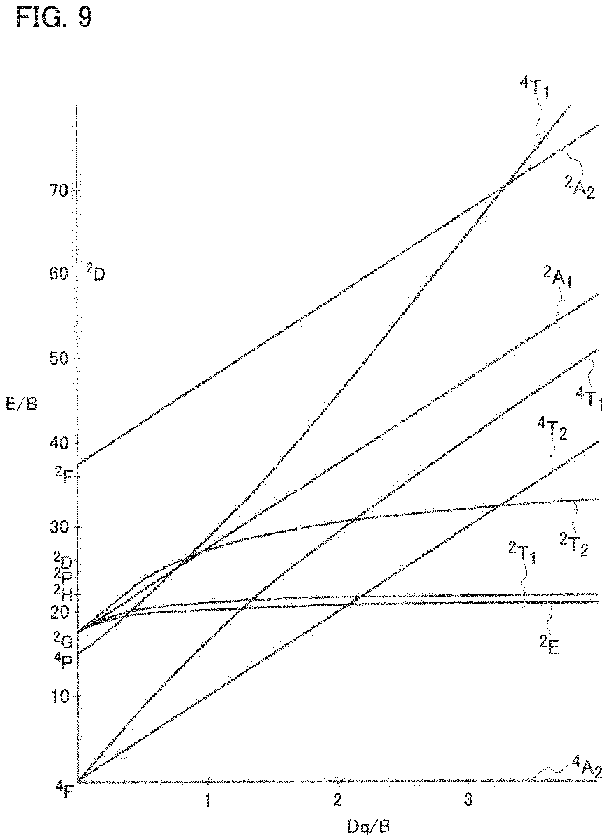

The electron energy transition of Cr³⁺ is described below. Fig. 9 illustrates electron energy levels of Cr³⁺. Specifically, Fig. 9 is a Tanabe-Sugano diagram applied to six-coordinated Cr³⁺, Mn⁴⁺, and the like.

The horizontal axis in Fig. 9 illustrates Dq, which represents the magnitude of a ligand-field splitting, divided by B of the Laker parameter, which represents the strength of an electrostatic repulsive force acting between electrons. The horizontal axis in Fig. 9 can be understood as an indicator of the strength of the ligand field that Cr³⁺ receives from surrounding ligands in a crystal. An example of ligands around Cr³⁺ in a crystal is oxygen ions.

The vertical axis in Fig. 9 illustrates an energy E from a ground state divided by B of the above-described Laker parameter. The vertical axis in Fig. 9 can be understood as an indicator of the magnitude of an electron energy of an excited state formed by three 3d electrons forming the outermost electron cloud of Cr³⁺, that is, an indicator indicating the energy difference between the excited state formed by the three 3d electrons and the ground state.

Fig. 9 illustrates that the electron energy of the excited state formed by 3d-orbital electrons of Cr³⁺ in a phosphor crystal takes several discrete states. Fig. 9 also illustrates that the state of the electron energy formed by electrons of Cr³⁺ in a phosphor crystal changes depending on the type, number, and arrangement of surrounding ligands, the distance to ligands, and the like, and consequently, the energy difference between the excited state and the ground state changes. Moreover, Fig. 9 illustrates that the electron energies in the excited state, which take several discrete states, behave differently depending on ligand fields. Note that symbols, such as ²E, ⁴T₂, and ⁴A₂, illustrated in Fig. 9 are known symbols indicating each of the discrete electron energy states formed by the three electrons in the 3d orbital of Cr³⁺.

Here, the electron energy transition accompanied by fluorescence usually results in an electron energy transition from the lowest excited state (²T₁ and ²E or ⁴T₂ in Fig. 9) to the ground state (⁴A₂ in Fig. 9). Thus, according to Fig. 9, when the strength of the ligand field received by Cr³⁺ in a crystal is strong (the value on the horizontal axis in Fig. 9 is large), Cr³⁺ exhibits fluorescence due to the electron energy transition from ²T₁ and ²E to ⁴A₂. Fig. 9 also illustrates that when the strength of the ligand field is weak (the value on the horizontal axis in Fig. 9 is small), Cr³⁺ exhibits fluorescence due to the electron energy transition from ⁴T₂ to ⁴A₂. The first phosphor 4 exhibits fluorescence due to the latter electron energy transition.

Note that in the electron energy transition from ²T₁ and ²E to ⁴A₂, as can be seen from Fig. 9, the fluorescence spectrum becomes linear because the energy difference does not change significantly even when the strength of the ligand field changes.

In contrast, in the electron energy transition from ⁴T₂ to ⁴A₂, as can be seen from Fig. 9, when the strength of the ligand field changes, the energy difference changes in a significant manner, and thus the fluorescence spectrum takes a broad shape. The fluorescence spectrum of the first phosphor 4 has a broad shape because it is based on the electron energy transition (spin-allowed transition) of ⁴T₂ → ⁴A₂.

Note that the energy transition between energy levels in the ²T₁ and ²E to ⁴A₂ electron energy transition of 3d electrons of Cr³⁺ is a parity-forbidden transition, and thus the fluorescence afterglow time is as long as 100 µs to less than 50 ms. The afterglow time of the fluorescence based on this Cr³⁺ is longer compared to that of Ce³⁺ or Eu²⁺ fluorescence (10 µs or less), which exhibit a parity-allowed transition. However, the electron energy transition from ⁴T₂ to ⁴A₂ of Cr³⁺ is a spin-allowed transition between two states having the same spin, and thus the afterglow time is relatively short, around 100 µs.

Such a Cr³⁺-activated phosphor that exhibits fluorescence due to a parity-forbidden (spin-allowed) electron energy transition exhibits a much longer afterglow property than an Eu²⁺-activated phosphor that exhibits fluorescence due to a parity-allowed electron energy transition. The present disclosure is achieved by finding that the Cr³⁺-activated phosphor exhibiting fluorescence due to a parity-forbidden electron energy transition has surprisingly little saturation of fluorescence output even though it exhibits a much longer afterglow property than an Eu²⁺-activated phosphor.

The first phosphor 4 emits fluorescence satisfying the following property (A) because the first wavelength-converted light 7 is fluorescence based on the spin-allowed electron energy transition of Cr³⁺. The first phosphor 4 preferably emits fluorescence that satisfies at least one of the properties (B) or (C), in addition to the property (A).

### [Property A]

The property (A) is that the fluorescence spectrum of the first wavelength-converted light 7 has a fluorescence intensity maximum value in a wavelength range exceeding 710 nm. Here, the fluorescence intensity maximum value means the maximum fluorescence intensity at a peak where the fluorescence intensity exhibits the maximum value between peaks in the fluorescence spectrum. The fluorescence spectrum of the first wavelength-converted light 7 preferably has a fluorescence intensity maximum value in a wavelength range of 710 nm or more and less than 900 nm.

With the light emitting device 1A satisfying the property (A) and the light irradiation type cosmetic apparatus 10 including the light emitting device 1A, in which the fluorescence spectrum of the first wavelength-converted light 7 has a fluorescence intensity maximum value in a wavelength range exceeding 710 nm, it is possible to easily obtain a point light source containing a large amount of the near-infrared component.

The light emitting device 1A satisfying the property (A) is suitable as a light emitting device included in the light irradiation type cosmetic apparatus 10 because the fluorescence spectrum of the first wavelength-converted light 7 has a fluorescence intensity maximum value in a wavelength range exceeding 710 nm, which is a wavelength range suitable for a light irradiation type cosmetic apparatus. The reason why the fluorescence spectrum of the first wavelength-converted light 7 is suitable for a light irradiation type cosmetic apparatus when it has a fluorescence intensity maximum value in a wavelength range exceeding 710 nm is described below.

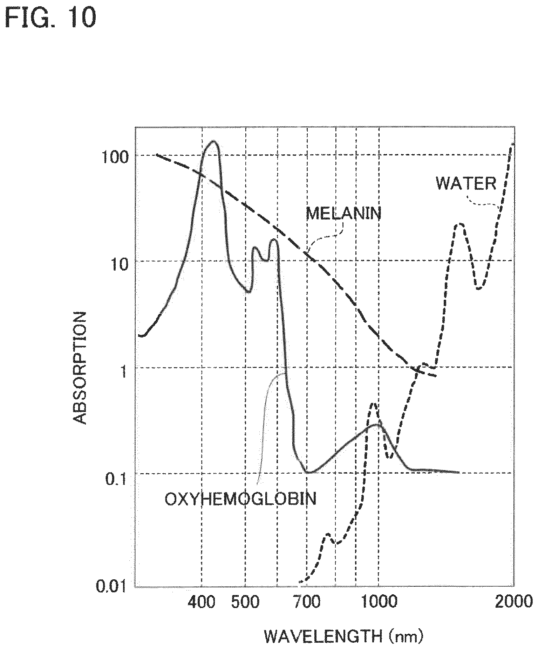

Fig. 10 illustrates a relationship between wavelengths of light and absorption rates of light in various substances. Specifically, Fig. 10 illustrates the relationship between wavelengths of light and absorption rates of light in oxyhemoglobin, melanin, and water.

Oxyhemoglobin is a substance contained in erythrocytes of a human and so on, and the light absorption rate of oxyhemoglobin can be regarded as the light absorption rate of human blood. Melanin is a substance contained in hair, spots, freckles, and the like of a human and so on, and the light absorption rate of melanin can be regarded as the light absorption rate of human hair, spots, and freckles. Water is a substance contained in a body fluid of a human and so on, and the light absorption rate of water can be regarded as the light absorption rate of human blood.

Thus, in Fig. 10, light in a wavelength range where the light absorption rate of melanin is low and that of oxyhemoglobin and water is high, such as light having a wavelength around 450 nm, is more easily absorbed by erythrocytes than melanin pigments. Such light has difficulties in suppressing hair growth and removing spots, freckles, and the like, tends to cause a burn on a person, and has a low thermal efficiency, which is not preferable for the light irradiation type cosmetic apparatus 10.

In contrast, in Fig. 10, light in a wavelength range where the light absorption rate of melanin is high and that of oxyhemoglobin and water is low, such as light having a wavelength exceeding 710 nm, is more easily absorbed by melanin pigments and is hardly absorbed by erythrocytes. That is, when such light is emitted on human skin or the like, heat is generated and thus the regenerative function of hair is decreased, resulting in a hair growth suppression effect. When such light is emitted on human skin or the like, heat is generated, resulting in destruction, coagulation, and the like of melanin pigments and the like, which constitute spots, freckles, and the like. Thus, the light in the wavelength range having a high light absorption rate of melanin and a low light absorption rate of oxyhemoglobin and water is preferable for the light irradiation type cosmetic apparatus 10 because the light facilitates hair growth suppression and removal of spots, freckles, and the like, is less likely to cause a burn on a person, and has a high thermal efficiency.

In Fig. 10, the above-described "light in the wavelength range having a high light absorption rate of melanin and a low light absorption rate of oxyhemoglobin and water" is light in a wavelength range generally exceeding 710 nm. Thus, when the fluorescence spectrum of the first wavelength-converted light 7 included in the output light 15 has a fluorescence intensity maximum value in a wavelength range exceeding 710 nm (satisfying the property (A)), it is possible to obtain the light irradiation type cosmetic apparatus 10 being useful for hair-growth suppression, and removal of spots, freckles, and the like.

### [Property (B)]

The property (B) is that the percentage of a fluorescence intensity at the wavelength of 780 nm with respect to the fluorescence intensity maximum value in the fluorescence spectrum of the first wavelength-converted light 7 exceeds 30%. Hereinafter, the above-described percentage of the fluorescence intensity is also referred to as a "780-nm fluorescence intensity percentage". The 780-nm fluorescence intensity percentage preferably exceeds 60%, more preferably exceeds 80%.

When the 780-nm fluorescence intensity percentage is within the above-described ranges, the first wavelength-converted light 7 contains a large amount of a fluorescent component in the near-infrared wavelength range (650 to 1000 nm) in which light easily penetrates a living body, which is called a "biological window". Thus, it is possible for the light irradiation type cosmetic apparatus 10 that emits the first wavelength-converted light 7 satisfying the property (B) to increase the light intensity of near-infrared light penetrating a living body.

### [Property C]

The property (C) is that the 1/10 afterglow of the first wavelength-converted light 7 is less than 1 ms. Here, the 1/10 afterglow means a time τ_{1/10} required from the time exhibiting the maximum emission intensity to the time to reach the 1/10 intensity of the maximum emission intensity. The 1/10 afterglow is preferably 10 µs or more and less than 1 ms, more preferably 10 µs or more and less than 800 µs, further more preferably 10 µs or more and less than 400 µs, particularly preferably 10 µs or more and less than 350 µs, more particularly preferably 10 µs or more and less than 100 µs.

When the 1/10 afterglow falls within the above-described ranges, the output of the fluorescence emitted by the first phosphor 4 is less likely to saturate, even when the power density of excitation light that excites the first phosphor 4 is high. It is thus possible for the light irradiation type cosmetic apparatus 10 that emits the first wavelength-converted light 7 satisfying the property (C) to emit high-output near-infrared light with less saturation of the fluorescence output when high-output density light emitted from a solid-state light source having a rated output of 1 W or more is emitted.

Note that the 1/10 afterglow of the first wavelength-converted light 7 becomes longer than that of short-afterglow (less than 10 µs) fluorescence based on the parity-allowed transition of Ce³⁺, Eu²⁺, or the like. This is because the first wavelength-converted light 7 is fluorescence based on the spin-allowed electron energy transition of Cr³⁺ and having a relatively long afterglow

As the first phosphor 4, it is possible to use phosphors such as Lu₂CaMg₂(SiO₄)₃:Cr³⁺, Y₃Ga₂(AlO₄)₃:Cr³⁺, Y₃Ga₂(GaO₄)₃:Cr³⁺, Gd₃Ga₂(AlO₄)₃:Cr³⁺, Gd₃Ga₂(GaO₄)₃:Cr³⁺, (Y, La)₃Ga₂(GaO₄)₃:Cr³⁺, (Gd, La)₃Ga₂(GaO₄)₃:Cr³⁺, Ca₂LuZr₂(AlO₄)₃:Cr³⁺, Ca₂GdZr₂(AlO₄)₃:Cr³⁺, Lu₃Sc₂(GaO₄)₃:Cr³⁺, Y₃Sc₂(AlO₄)₃:Cr³⁺, Y₃Sc₂(GaO₄)₃:Cr³⁺, Gd₃Sc₂(GaO₄)₃:Cr³⁺, La₃Sc₂(GaO₄)₃:Cr³⁺, Ca₃Sc₂(SiO₄)₃:Cr³⁺, Ca₃Sc₂(GeO₄)₃:Cr³⁺, BeAl₂O₄:Cr³⁺, LiAl₅O₈:Cr³⁺, LiGa₅O₈:Cr³⁺, Mg₂SiO₄:Cr³⁺, Li⁺, La₃Ga₅GeO₁₄:Cr³⁺, and La₃Ga_{5.5}Nb_{0.5}O₁₄:Cr³⁺.

The first phosphor 4 is preferably made from ceramic. When the first phosphor 4 is made from ceramic, the heat dissipation of the first phosphor 4 is enhanced, and thus a decrease in the output of the first phosphor 4 due to temperature quenching is suppressed, and it becomes possible for the light irradiation type cosmetic apparatus 10 to emit high-output near-infrared light.

In the light irradiation type cosmetic apparatus 10, the first wavelength-converted light 7 emitted by the first phosphor 4 has a specific fluorescent component based on the electron energy transition of Cr^{3 +}.

Note that the fluorescence spectrum of the first wavelength-converted light 7 preferably does not include traces of a linear spectral component derived from the electron energy transition of Cr³⁺. The linear spectral component derived from the electron energy transition of Cr³⁺ is a long-afterglow fluorescent component due to the spin-forbidden transition of Cr³⁺. When the fluorescence spectrum of the first wavelength-converted light 7 does not include the above-described traces, the first wavelength-converted light 7 does not contain a long-afterglow fluorescent component due to the spin-forbidden transition of Cr³⁺. It is thus possible to obtain a high-output point light source having less saturation of fluorescence output when the light irradiation type cosmetic apparatus 10 emits light having high-power density that is emitted from a solid-state light source having a rated output of 1 W or more.

The wavelength converter 3A includes only the first phosphor 4 that contains fluorescence based on the electron energy transition of Cr³⁺, as a phosphor. The first phosphor 4 contains no activator other than Cr³⁺. Thus, light absorbed by the first phosphor 4 is converted into only the fluorescence based on the electron energy transition of Cr³⁺. Thus, the light irradiation type cosmetic apparatus 10 in which the first phosphor 4 contains no activator other than Cr³⁺ facilitates the design of output light to maximize the output proportion of the near-infrared fluorescent component.

The first phosphor 4 preferably has a garnet crystal structure. The ease of compositional deformation of the garnet phosphor enables the preparation of numerous phosphor compounds. Thus, when the first phosphor 4 has a garnet crystal structure, it is easy to adjust a crystal field around Cr³⁺ and to control the color tone of fluorescence based on the electron energy transition of Cr³⁺.

Note that a phosphor having a garnet structure, especially an oxide, has a polyhedral particle shape close to a sphere and has excellent dispersibility in a phosphor particle group. Thus, when the first phosphor 4 has a garnet structure, it is relatively easy to manufacture the wavelength converter 3A having excellent optical transparency, which makes it possible to achieve high output of the obtained light irradiation type cosmetic apparatus 10. Since a phosphor with a garnet crystal structure has a record of practical application as a phosphor for LEDs, the light irradiation type cosmetic apparatus 10 in which the first phosphor 4 has a garnet crystal structure is highly reliable.

The first phosphor 4 is preferably an oxide-based phosphor, more preferably an oxide phosphor. Note that the oxide-based phosphor refers to a phosphor containing oxygen but not nitrogen.

An oxide is a stable substance in the atmosphere, and thus when an oxide phosphor is heated through high density photoexcitation with light emitted from a solid-state light source having a rated output of 1 W or more, the change in quality of a phosphor crystal due to oxidation in the atmosphere is less likely to occur compared to a nitride phosphor. When the first phosphor 4 is entirely an oxide-based phosphor, it is possible to obtain the light irradiation type cosmetic apparatus 10 having high reliability.

Note that the first phosphor 4 may contain two or more kinds of Cr³⁺-activated phosphors. When the first phosphor 4 contains two or more kinds of Cr³⁺-activated phosphors, it is possible to control at least the output light component in the near-infrared wavelength range. Thus, in the light irradiation type cosmetic apparatus 10 in which the first phosphor 4 contains two or more kinds of Cr³⁺-activated phosphors, it becomes easy to adjust the spectral distribution of the near-infrared fluorescent component.

### <Sealant>

In the wavelength converter 3A, the first phosphor 4 is contained in the sealant 5. Preferably, the first phosphor 4 is dispersed in the sealant 5. When the first phosphor 4 is dispersed in the sealant 5, it becomes possible to efficiently absorb the primary light 6 emitted by the light source 2 and efficiently convert the wavelength thereof to that of near-infrared light. When the first phosphor 4 is dispersed in the sealant 5, the wavelength converter 3A can be easily formed into a sheet or film shape.

The sealant 5 is made from at least one of an organic material or an inorganic material. The sealant 5 is preferably made from at least one of a transparent (translucent) organic material or a transparent (translucent) inorganic material. An example of the organic material sealant is a transparent organic material, such as a silicone resin. An example of the inorganic material sealant is a transparent inorganic material, such as low melting point glass.

Note that the wavelength converter 3A is preferably made from an inorganic material. An inorganic material here means a material other than an organic material, and includes ceramics and metals as a concept. When the wavelength converter 3A is made from an inorganic material, the thermal conductivity thereof is higher than that of the wavelength converter containing an organic material such as a sealing resin, which facilitates the design of heat dissipation. Thus, even when the first phosphor 4 is photoexcited at high density by the primary light 6 emitted by the light source 2, it is possible to suppress the temperature rise of the wavelength converter 3A in an effective manner. Consequently, the temperature quenching of the first phosphor 4 in the wavelength converter 3A is suppressed, which makes it possible to achieve high output of emitted light.

When the wavelength converter 3A is made from an inorganic material, the sealant 5 is preferably made from an inorganic material. The inorganic material for the sealant 5 is preferably zinc oxide (ZnO). When the sealant 5 is made from an inorganic material, the heat dissipation of the first phosphor 4 is further enhanced, and thus a decrease in output of the first phosphor 4 due to temperature quenching is suppressed, which makes it possible to emit high-output near-infrared light.

Note that as a modified example of the light irradiation type cosmetic apparatus 10, it is possible to replace the wavelength converter 3A with a wavelength converter that does not include the sealant 5. In this case, particles of the first phosphor 4 can be fixed to each other using an organic or inorganic binder. It is also possible to fix particles of the first phosphor 4 to each other through a heating reaction of the first phosphor 4. A generally used resin-based adhesive, ceramics fine particles, low-melting-point glass, or the like can be used as the binder. In the wavelength converter without the sealant 5, it is possible to reduce the thickness of the wavelength converter.

### <Action>

The action of the light irradiation type cosmetic apparatus 10 including the light emitting device 1A will be described. First, the primary light 6 emitted from the light source 2 of the light emitting device 1A is emitted on a front 3a of the wavelength converter 3A. The emitted primary light 6 penetrates the wavelength converter 3A. When the primary light 6 penetrates the wavelength converter 3A, the first phosphor 4 included in the wavelength converter 3A absorbs part of the primary light 6 and emits the first wavelength-converted light 7. In this way, the output light 15 including the primary light 6 and the first wavelength-converted light 7 is emitted from a back 3b of the wavelength converter 3A.

The output light 15 emitted from the light emitting device 1A is emitted to the outside from the irradiation port 110 of the light irradiation type cosmetic apparatus 10. When the output light 15 is emitted on human skin from the irradiation port 110 of the light irradiation type cosmetic apparatus 10, the output light 15 acts on the human skin to provide a hair growth suppression effect, an effect of the removal of spots, freckles, and the like. Specifically, the first wavelength-converted light 7 contained in the output light 15 is light that emits fluorescence based on the electron energy transition of Cr³⁺ and satisfying the above-described property (A) and contains a light component in a wavelength range suitable for easier absorption by melanin pigments than by blood and the like. Thus, when the output light 15 including the first wavelength-converted light 7 is emitted on human skin, a burn is less likely to occur, thermal efficiency is high, and destruction, coagulation, or the like occurs in melanin pigments and the like. When the melanin pigments and the like, which have been destroyed or coagulated, come to the surface through the skin's metabolism and are then peeled off and removed from the skin, it is expected that beautiful skin can be obtained through the suppression of unwanted hair growth and the removal of spots, freckles, and the like.

The light emitting device 1A and the light irradiation type cosmetic apparatus 10 including the light emitting device 1A are capable of increasing the intensity of near-infrared light penetrating a living body through the "biological window", and thus have a high effect in suppressing unwanted hair growth, high performance in removing spots and freckles, and the like.

The first wavelength-converted light 7 contained in the output light 15 of the light emitting device 1A and the light irradiation type cosmetic apparatus 10 including the light emitting device 1A is fluorescence based on the spin-allowed electron energy transition of Cr³⁺ of the first phosphor 4. Thus, with the light emitting device 1A and the light irradiation type cosmetic apparatus 10 including the light emitting device 1A, output light design that maximizes the output proportion of the near-infrared fluorescent component is easy, and thus filtering and removing extra light components is unnecessary and the energy efficiency is high.

### (More specific examples of light emitting device (second to fifth light emitting devices))

In the above-described light irradiation type cosmetic apparatus 10 illustrated in Figs. 1 to 3, both the light source 2 and the wavelength converter 3A constituting the light emitting device 1A are provided in the body part 20. However, one or both of the light source 2 and the wavelength converter 3 constituting the light emitting device 1 can be provided in the head part 30, as a configuration of a modified example of the light irradiation type cosmetic apparatus 10

For example, it is possible to provide the light source 2 in the head part 30 and provide the wavelength converter 3 in the body part 20, as a configuration of a modified example of the light irradiation type cosmetic apparatus 10. This modified example of the light irradiation type cosmetic apparatus 10 has a configuration in which, usually, the primary light 6 is emitted from the light source 2 in the head part 30 to the wavelength converter 3 in the body part 20 and the output light 15 is emitted from the wavelength converter 3 toward the head part 30. The output light 15 emitted from the wavelength converter 3 toward the head part 30 usually includes the first wavelength-converted light 7 emitted from the wavelength converter 3 and the primary light 6 reflected by the wavelength converter 3. Thus, the light irradiation type cosmetic apparatus 10 and its modified example have multiple aspects in which the arrangements of the light source 2 and the wavelength converter 3 constituting the light emitting device 1 are different.

The wavelength converter 3A of the first light emitting device 1A includes at least a first phosphor as a phosphor and includes a phosphor other than the first phosphor, as necessary. Specifically, the wavelength converter 3A of the first light emitting device 1A can have one aspect in which only the first phosphor is included as a phosphor, and another aspect in which the first phosphor and a phosphor other than the first phosphor are included as phosphors. As described above, the light irradiation type cosmetic apparatus 10 and its modified example have multiple aspects in which the types of phosphors included in the wavelength converter 3 are different.

As described above, the first light emitting device 1A has multiple aspects in which the arrangements of the light source 2 and the wavelength converter 3 constituting the light emitting device 1 are different, and/or the types of phosphors included in the wavelength converter 3 are different. The following is a description of more specific examples of the light emitting device (second to fifth light emitting devices) 1B to 1E in which the arrangements of the light source 2 and the wavelength converter 3 of the first light emitting device 1A, and the types of phosphors included in the wavelength converter 3 are described more specifically.

Note that the second to fifth light emitting devices 1B to 1E are more specific examples of the first light emitting device 1A, and thus the first light emitting device 1A and the second to fifth light emitting devices 1B to 1E have the same basic configuration. Thus, in the following description of the second to fifth light emitting devices 1B to 1E, the first light emitting device 1A may also be referred to as appropriate.

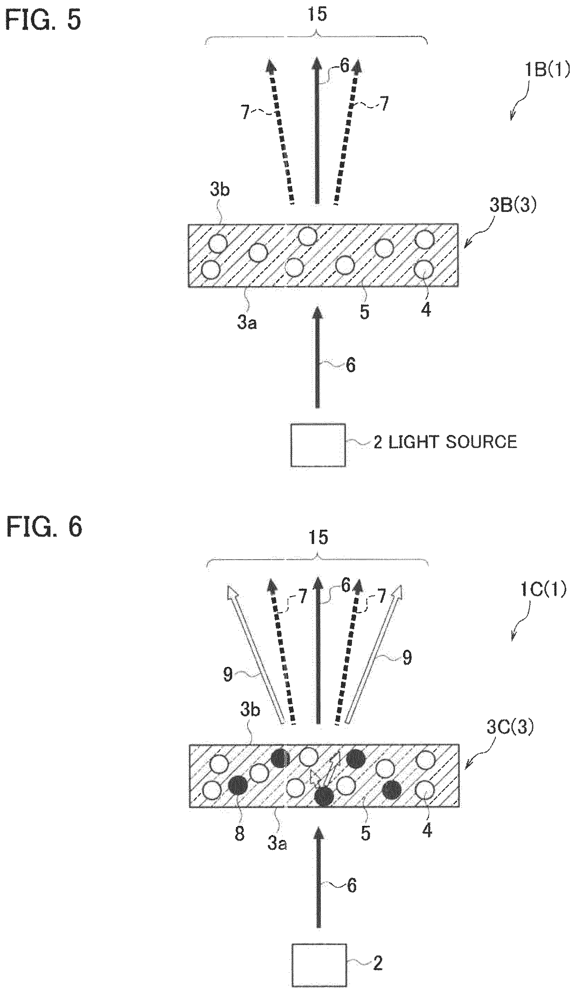

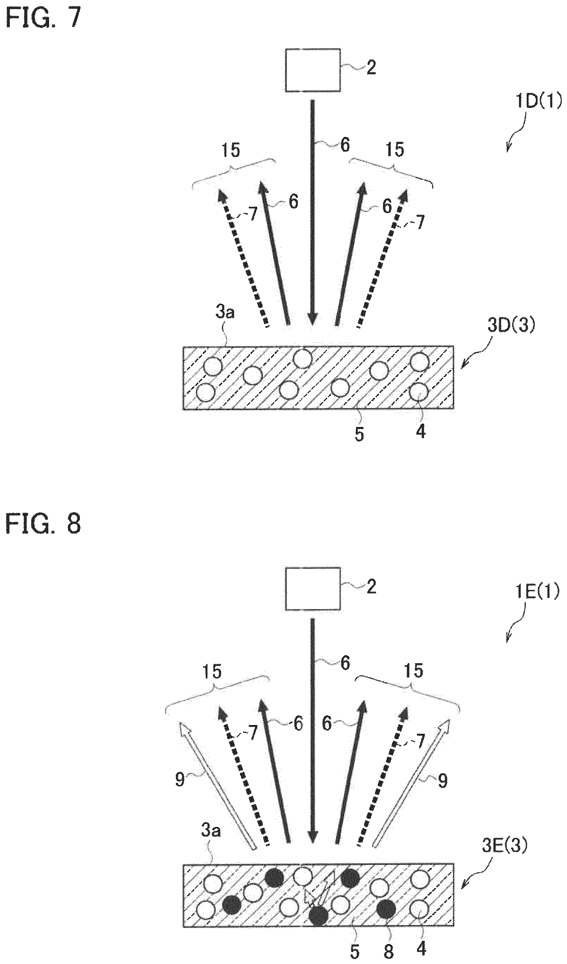

Fig. 5 is a schematic cross-sectional view of a more specific example of the light emitting device (second light emitting device) included in the light irradiation type cosmetic apparatus. Fig. 6 is a schematic cross-sectional view of a more specific example of the light emitting device (third light emitting device) included in the light irradiation type cosmetic apparatus. Fig. 7 is a schematic cross-sectional view illustrating a more specific example of the light emitting device (fourth light emitting device) included in the light irradiation type cosmetic apparatus. Fig. 8 is a schematic cross-sectional view illustrating a more specific example of the light emitting device (fifth light emitting device) included in the light irradiation type cosmetic apparatus.

The second light emitting device 1B illustrated in Fig. 5 includes a wavelength converter 3B (3) containing only the first phosphor 4 as a phosphor and generates the output light 15 using the first wavelength-converted light 7 and the primary light 6 that has penetrated the wavelength converter 3B.

The third light emitting device 1C illustrated in Fig. 6 includes a wavelength converter 3C (3) containing the first phosphor 4 and a second phosphor 8 as phosphors and generates the output light 15 using the first wavelength-converted light 7, a second wavelength-converted light 9, and the primary light 6 that has penetrated the wavelength converter 3C.

The fourth light emitting device 1D illustrated in Fig. 7 includes a wavelength converter 3D (3) containing only the first phosphor 4 as a phosphor and generates the output light 15 using the first wavelength-converted light 7 and the primary light 6 that has been reflected by the wavelength converter 3D.

The fifth light emitting device 1E illustrated in Fig. 8 includes a wavelength converter 3E (3) containing the first phosphor 4 and the second phosphor 8 as phosphors and generates the output light 15 using the first wavelength-converted light 7, the second wavelength-converted light 9, and the primary light 6 that has been reflected by the wavelength converter 3E.

The wavelength converter 3B of the second light emitting device 1B illustrated in Fig. 5 and the wavelength converter 3C of the third light emitting device 1C illustrated in Fig. 6 are configured to receive the primary light 6 at the front 3a, which is the surface facing the light source 2, and to emit fluorescence from the back 3b, which is the surface on the opposite side to the surface facing the light source 2. The wavelength converter 3D of the fourth light emitting device 1D illustrated in Fig. 7 and the wavelength converter 3E of the fifth light emitting device 1E illustrated in Fig. 8 are configured to receive the primary light 6 at the front 3a and emit fluorescence from the same front 3a. The second to fifth light emitting devices 1B to 1E are specifically described below.

### (Light emitting device (second light emitting device))

The light emitting device (second light emitting device) 1B includes the wavelength converter 3B (3) containing only the first phosphor 4 as a phosphor and generates the output light 15 using the first wavelength-converted light 7 and the primary light 6 that has penetrated the wavelength converter 3B.

For the second light emitting device 1B, the wavelength converter 3A of the first light emitting device 1A is described more specifically, and other configurations are simplified. The main difference between the second light emitting device 1B and the first light emitting device 1A is only in the wavelength converter 3B. Thus, the wavelength converter 3B is described below, and for other members, the description of their configurations and actions is omitted or simplified.

### <Wavelength converter>

The wavelength converter 3B includes the first phosphor 4 and the sealant 5. In the wavelength converter 3B, the first phosphor 4 is included in the sealant 5.

### <Action>

The action of the light irradiation type cosmetic apparatus 10 including the light emitting device 1B will be described. First, the primary light 6 emitted from the light source 2 of the light emitting device 1B is emitted on the front 3a of the wavelength converter 3B. The emitted primary light 6 penetrates the wavelength converter 3B. When the primary light 6 penetrates the wavelength converter 3B, the first phosphor 4 included in the wavelength converter 3B absorbs part of the primary light 6 and emits the first wavelength-converted light 7. In this way, the output light 15 including the primary light 6 and the first wavelength-converted light 7 is emitted from the back 3b of the wavelength converter 3B.

The output light 15 emitted from the light emitting device 1B is emitted to the outside from the irradiation port 110 of the light irradiation type cosmetic apparatus 10. When the output light 15 is emitted on human skin from the irradiation port 110 of the light irradiation type cosmetic apparatus 10, the output light 15 acts on the human skin to provide a hair growth suppression effect, and an effect of the removal of spots, freckles, and the like. The action of the output light 15 acting on the human skin to provide the hair growth suppression effect, and the effect of the removal of spots, freckles, and the like is the same as that of the light irradiation type cosmetic apparatus 10 including the light emitting device 1A, and thus the description is omitted.

The light emitting device 1B and the light irradiation type cosmetic apparatus 10 including the light emitting device 1B are capable of increasing the intensity of near-infrared light penetrating a living body through the "biological window", and thus have a high effect in suppressing unwanted hair growth, high performance in removing spots and freckles, and the like.

### (Light emitting device (third light emitting device))

The light emitting device (third light emitting device) 1C includes the wavelength converter 3C (3) containing the first phosphor 4 and the second phosphor 8 as phosphors and generates the output light 15 using the first wavelength-converted light 7, the second wavelength-converted light 9, and the primary light 6 that has penetrated the wavelength converter 3C.

The third light emitting device 1C uses the wavelength converter 3C instead of the wavelength converter 3B of the second light emitting device 1B. The difference between the third light emitting device 1C and the second light emitting device 1B is only in the wavelength converter 3C. Thus, the wavelength converter 3C is described below, and for other members, the description of their configurations and actions is omitted or simplified.

### <Wavelength converter>

The wavelength converter 3C includes the first phosphor 4, the second phosphor 8, and the sealant 5. In the wavelength converter 3C, the first phosphor 4 and the second phosphor 8 are included in the sealant 5. That is, the wavelength converter 3C of the third light emitting device 1C further includes the second phosphor 8 that absorbs the primary light 6 and converts it into the second wavelength-converted light 9 having a wavelength longer than that of the primary light 6 and being different from the first wavelength-converted light 7.

The wavelength converter 3C is the same as the wavelength converter 3B of the second light emitting device 1B except that the wavelength converter 3C further includes the second phosphor 8. Thus, the second phosphor 8 is mainly described below, and the description of other configurations and actions is omitted or simplified.

### [Second phosphor]

The second phosphor 8 absorbs the primary light 6 and converts it into the second wavelength-converted light 9 having a wavelength longer than that of the primary light 6 and being different from the first wavelength-converted light 7. In the third light emitting device 1C, the wavelength converter 3C further includes the second phosphor 8 in addition to the first phosphor 4, and thus it is possible to emit white output light using additive mixing with the primary light 6 emitted by the light source 2, which is warm color light, for example.

As described above, when the wavelength converter 3C of the fifth light emitting device 1C further includes the second phosphor 8 in addition to the first phosphor 4, it becomes possible to control the shape of the fluorescence spectrum emitted by the wavelength converter 3C and the excitation properties. Thus, it is possible for the obtained light emitting device 1C and the light irradiation type cosmetic apparatus 10 including the light emitting device 1C to easily adjust the spectral distribution of the output light depending on use.

The second phosphor 8 included in the wavelength converter 3C is not limited as long as it can absorb the primary light 6 emitted by the light source 2 and emit the second wavelength-converted light 9 that is visible light. The second phosphor 8 is preferably a Ce³⁺-activated phosphor having, as a host, a compound having, as a main component, at least one selected from the group of compounds consisting of garnet-type, calcium ferrite-type, and lanthanum silicon nitride (La₃Si₆N₁₁)-type crystal structures. The second phosphor 8 is preferably a Ce³⁺-activated phosphor having, as a host, at least one compound selected from the group of compounds consisting of garnet-type, calcium-ferrite-type, and lanthanum silicon nitride (La₃Si₆N₁₁)-type crystal structures. When the above-described second phosphor 8 is used, it becomes possible to obtain output light having a large amount of light components from green-based to yellow-based light components.

As the second phosphor 8, for example, a Ce³⁺-activated phosphor having, as a host, a compound (B) having, as a main component, at least one selected from the group consisting of M₃RE₂(SiO₄)₃, RE₃Al₂(AlO₄)₃, MRE₂O₄, and RE₃Si₆N₁₁ is used. As the second phosphor 8, for example, a Ce³⁺-activated phosphor having, as a host, at least one selected from the group consisting of M₃RE₂(SiO₄)₃, RE₃Al₂(AlO₄)₃, MRE₂O₄, and RE₃Si₆N₁₁ is used. The second phosphor 8 is preferably a Ce³⁺-activated phosphor having, as a host, a solid solution having the above-described compound (B) as an end component. Note that in the compound (B), M is an alkaline earth metal, and RE is a rare earth element.

The above-described second phosphor 8 well absorbs light within a wavelength range of 430 nm or more and 480 nm or less and converts it with high efficiency to green to yellow-based light having the intensity maximum value within a wavelength range of 540 nm or more and less than 590 nm. Thus, by using the light source 2 that emits warm color light as the primary light 6 and using the second phosphor 8, it is possible to easily obtain a visible light component.

When the wavelength converter 3C includes the first phosphor 4 and the second phosphor 8, the first phosphor 4 preferably emits the first wavelength-converted light 7 by absorbing at least one of the primary light 6 emitted by the light source 2 or the second wavelength-converted light 9 emitted by the second phosphor 8. As described above, the first phosphor 4 is preferably a phosphor that absorbs the primary light 6 emitted by the light source 2 and emits the first wavelength-converted light 7 that is near-infrared light.

The first phosphor 4 may be a phosphor that absorbs the second wavelength-converted light 9 emitted by the second phosphor 8 and emits the first wavelength-converted light 7 that is near-infrared light. That is, the second phosphor 8 may be excited by the primary light 6 to emit the second wavelength-converted light 9, and the first phosphor 4 may be excited by the second wavelength-converted light 9 to emit the first wavelength-converted light 7. Here, even when the first phosphor 4 is a phosphor hardly excited by the primary light 6, it becomes possible to excite the first phosphor 4 through the second phosphor 8 by using fluorescence emitted by the second phosphor 8.

For this reason, when the first phosphor 4 absorbs the second wavelength-converted light 9 and emits the first wavelength-converted light 7, it becomes possible to select as the first phosphor 4 a phosphor that absorbs visible light. Thus, when the first phosphor 4 absorbs the second wavelength-converted light 9 and emits the first wavelength-converted light 7, the choice of the first phosphor 4 expands, and the industrial production of the light emitting device 1C and the light irradiation type cosmetic apparatus 10 including the light emitting device 1C becomes easy. When the first phosphor 4 absorbs the second wavelength-converted light 9 and emits the first wavelength-converted light 7, it becomes possible for the light emitting device 1C and the light irradiation type cosmetic apparatus 10 including the light emitting device 1C to emit the first wavelength-converted light 7 having a large near-infrared light component intensity.

Note that the second phosphor 8 may contain two or more kinds of Cr³⁺ -activated phosphors. When the second phosphor 8 contains two or more kinds of Cr³⁺-activated phosphors, it is possible to control at least the output light component in the near-infrared wavelength range, which makes it easy to adjust the spectral distribution of the near-infrared fluorescent component.

### <Action>

The action of the light irradiation type cosmetic apparatus 10 including the light emitting device 1C will be described. First, the primary light 6 emitted from the light source 2 is emitted on the front 3a of the wavelength converter 3C. The emitted primary light 6 penetrates the wavelength converter 3C. When the primary light 6 penetrates the wavelength converter 3C, the second phosphor 8 included in the wavelength converter 3C absorbs part of the primary light 6 and emits the second wavelength-converted light 9. Furthermore, the first phosphor 4 included in the wavelength converter 3C absorbs the primary light 6 and/or part of the second wavelength-converted light 9 to emit the first wavelength-converted light 7. In this way, the output light 15 including the primary light 6, the first wavelength-converted light 7, and the second wavelength-converted light 9 is emitted from the back 3b of the wavelength converter 3C.

The output light 15 emitted from the light emitting device 1C is emitted to the outside from the irradiation port 110 of the light irradiation type cosmetic apparatus 10. When the output light 15 is emitted on human skin from the irradiation port 110 of the light irradiation type cosmetic apparatus 10, the output light 15 acts on the human skin to provide a hair growth suppression effect, an effect of the removal of spots, freckles, and the like. The action of the output light 15 acting on the human skin to provide the hair growth suppression effect, and the effect of the removal of spots, freckles, and the like is the same as that of the light irradiation type cosmetic apparatus 10 including the light emitting device 1A, and thus the description is omitted.

The light emitting device 1C and the light irradiation type cosmetic apparatus 10 including the light emitting device 1C are capable of increasing the intensity of near-infrared light penetrating a living body through the "biological window", and thus have a high effect in suppressing unwanted hair growth, high performance in removing spots and freckles, and the like.

### (Light emitting device (fourth light emitting device))

The light emitting device (fourth light emitting device) 1D includes the wavelength converter 3D (3) containing only the first phosphor 4 as a phosphor and generates the output light 15 using the first wavelength-converted light 7 and the primary light 6 that has been reflected by the wavelength converter 3D.

The fourth light emitting device 1D uses the wavelength converter 3D instead of the wavelength converter 3B of the second light emitting device 1B. The difference between the fourth light emitting device 1D and the second light emitting device 1B is only in the wavelength converter 3D. Thus, the wavelength converter 3D is described below, and for other members, the description of their configurations and actions is omitted or simplified.

### <Wavelength converter>

The wavelength converter 3D includes the first phosphor 4 and the sealant 5. In the wavelength converter 3D, the first phosphor 4 is included in the sealant 5. The wavelength converter 3D is the same as the wavelength converter 3B of the second light emitting device 1B in that it includes the first phosphor 4 and the sealant 5, but the wavelength converter 3D has an optical action different from that of the wavelength converter 3B.

In the wavelength converter 3B of the second light emitting device 1B, the primary light 6 emitted on the wavelength converter 3B penetrates the wavelength converter 3B. In contrast, in the wavelength converter 3D of the fourth light emitting device 1D, most of the primary light 6 emitted on the wavelength converter 3D enters the wavelength converter 3D from the front 3a of the wavelength converter 3D, and the remainder is reflected by the front 3a.

The wavelength converter 3D is configured in such a manner that the emitted light of the primary light 6 enters from the front 3a of the wavelength converter 3D, and the output light of the first phosphor 4 is emitted from the front 3a of the wavelength converter 3D. Thus, most of the primary light 6 emitted on the wavelength converter 3D enters the wavelength converter 3D from the front 3a of the wavelength converter 3D, and the remainder is reflected by the front 3a.

### <Action>

The action of the light irradiation type cosmetic apparatus 10 including the light emitting device 1D will be described. First, the primary light 6 emitted from the light source 2 is emitted on the front 3a of the wavelength converter 3D. Most of the primary light 6 enters the wavelength converter 3D from the front 3a of the wavelength converter 3D, and the remainder is reflected by the front 3a. In the wavelength converter 3D, the first wavelength-converted light 7 is emitted from the first phosphor 4 excited by the primary light 6, and the first wavelength-converted light 7 is emitted from the front 3a. In this way, the output light 15 including the primary light 6 and the first wavelength-converted light 7 is emitted from the front 3a of the wavelength converter 3D.

The output light 15 emitted from the light emitting device 1D is emitted to the outside from the irradiation port 110 of the light irradiation type cosmetic apparatus 10. When the output light 15 is emitted on human skin from the irradiation port 110 of the light irradiation type cosmetic apparatus 10, the output light 15 acts on the human skin to provide a hair growth suppression effect, and an effect of the removal of spots, freckles, and the like. The action of the output light 15 acting on the human skin to provide the hair growth suppression effect, and the effect of the removal of spots, freckles, and the like is the same as that of the light irradiation type cosmetic apparatus 10 including the light emitting device 1A, and thus the description is omitted.

The light emitting device 1D and the light irradiation type cosmetic apparatus 10 including the light emitting device 1D are capable of increasing the intensity of near-infrared light penetrating a living body through the "biological window", and thus have a high effect in suppressing unwanted hair growth, high performance in removing spots and freckles, and the like.

### (Light emitting device (fifth light emitting device))

The light emitting device (fifth light emitting device) 1E includes the wavelength converter 3E (3) containing the first phosphor 4 and the second phosphor 8 as phosphors and generates the output light 15 using the first wavelength-converted light 7, the second wavelength-converted light 9, and the primary light 6 that has been reflected by the wavelength converter 3E.

The fifth light emitting device 1E uses the wavelength converter 3E instead of the wavelength converter 3C of the third light emitting device 1C. The difference between the fifth light emitting device 1E and the third light emitting device 1C is only in the wavelength converter 3E. Thus, the wavelength converter 3E is described below, and for other members, the description of their configurations and actions is omitted or simplified.

### <Wavelength converter>

The wavelength converter 3E includes the first phosphor 4, the second phosphor 8, and the sealant 5. In the wavelength converter 3E, the first phosphor 4 and the second phosphor 8 are included in the sealant 5. That is, the wavelength converter 3E of the light emitting device 1E further includes the second phosphor 8 that absorbs the primary light 6 and converts it into the second wavelength-converted light 9 having a wavelength longer than that of the primary light 6 and being different from the first wavelength-converted light 7. The wavelength converter 3E is the same as the wavelength converter 3C of the third light emitting device 1C in that it includes the first phosphor 4, the second phosphor 8, and the sealant 5, but has an optical action different from that of the wavelength converter 3C.

The second phosphor 8 used in the wavelength converter 3E is the same as the wavelength converter 3C of the third light emitting device 1C, and thus the description is omitted. In the fifth light emitting device 1E, the wavelength converter 3E includes the second phosphor 8, and thus it is possible to emit white output light using additive mixing with the primary light 6 emitted by the light source 2, which is warm color light, for example.

As described above, when the first phosphor 4 and the second phosphor 8 are appropriately combined and used, it becomes possible to control the shape of the fluorescence spectrum of the first wavelength-converted light 7 and the excitation properties. That is, when the wavelength converter 3E of the fifth light emitting device 1E further includes the second phosphor 8 in addition to the first phosphor 4, it becomes possible to control the shape of the fluorescence spectrum of the first wavelength-converted light 7 and the excitation properties. Thus, it is possible for the obtained light emitting device 1E and the light irradiation type cosmetic apparatus 10 including the light emitting device 1E to easily adjust the spectral distribution of the output light depending on use.

In the wavelength converter 3C of the third light emitting device 1C, the primary light 6 emitted on the wavelength converter 3C penetrates the wavelength converter 3C. In contrast, in the wavelength converter 3E of the fifth light emitting device 1E, most of the primary light 6 emitted on the wavelength converter 3E enters the wavelength converter 3E from the front 3a of the wavelength converter 3E, and the remainder is reflected by the front 3a.

In the wavelength converter 3E, the emitted light of the primary light 6 enters from the front 3a of the wavelength converter 3E, and the output light of the first phosphor 4 is emitted from the front 3a of the wavelength converter 3E. Thus, most of the primary light 6 emitted on the wavelength converter 3E enters the wavelength converter 3E from the front 3a of the wavelength converter 3E, and the remainder is reflected by the front 3a.

### <Action>

The action of the light irradiation type cosmetic apparatus 10 including the light emitting device 1E will be described. First, the primary light 6 emitted from the light source 2 is emitted on the front 3a of the wavelength converter 3E. Most of the primary light 6 enters the wavelength converter 3E from the front 3a of the wavelength converter 3E, and the remainder is reflected by the front 3a. In the wavelength converter 3E, the second wavelength-converted light 9 is emitted from the second phosphor 8 excited by the primary light 6, and the first wavelength-converted light 7 is emitted from the first phosphor 4 excited by the primary light 6 and/or the second wavelength-converted light 9. Then, the first wavelength-converted light 7 and the second wavelength-converted light 9 are emitted from the front 3a. In this way, the output light 15 including the primary light 6, the first wavelength-converted light 7, and the second wavelength-converted light 9 is emitted from the front 3a of the wavelength converter 3E.

The output light 15 emitted from the light emitting device 1E is emitted to the outside from the irradiation port 110 of the light irradiation type cosmetic apparatus 10. When the output light 15 is emitted on human skin from the irradiation port 110 of the light irradiation type cosmetic apparatus 10, the output light 15 acts on the human skin to provide a hair growth suppression effect, and an effect of the removal of spots, freckles, and the like. The action of the output light 15 acting on the human skin to provide the hair growth suppression effect, and the effect of the removal of spots, freckles, and the like is the same as that of the light irradiation type cosmetic apparatus 10 including the light emitting device 1A, and thus the description is omitted.

The light emitting device 1E and the light irradiation type cosmetic apparatus 10 including the light emitting device 1E are capable of increasing the intensity of near-infrared light penetrating a living body through the "biological window", and thus have a high effect in suppressing unwanted hair growth, high performance in removing spots and freckles, and the like.

### [Electronic device]

An electronic device according to the present embodiment can be obtained using the light irradiation type cosmetic apparatus 10 including any one of the above-described light emitting devices 1A to 1E. That is, the electronic device according to the present embodiment is provided with the light irradiation type cosmetic apparatus 10 including any one of the above-described light emitting devices 1A to 1E. An example of such an electronic device includes one provided with the above-described light emitting device 1 (1A to 1E) and a fiber (light guide member) that guides the output light 15 emitted from the light emitting device 1.

### EXAMPLES

The present embodiment is described in more detail below with examples and comparative examples, but the present embodiment is not limited to these examples.

### [Example 1]

### (Preparation of phosphor)

An oxide phosphor was synthesized through a preparation procedure using a solid-state reaction. Specifically, an oxide phosphor expressed by the composition formula of Y₃(Ga_{0.98}, Cr_{0.02})₂(GaO₄)₃ was synthesized. Note that the following compound powders were used as main raw materials in synthesizing the oxide phosphor.

Yttrium oxide (Y₂O₃): purity 3N, manufactured by Shin-Etsu Chemical Co., Ltd.

Gallium oxide (Ga₂O₃): purity 4N, manufactured by Nippon Rare Metal, Inc.

Chromium oxide (Cr₂O₃): purity 3N, manufactured by Kojundo Chemical Lab. Co., Ltd.

First, the above raw materials were weighed to give a compound Y₃(Ga_{0.98}, Cr_{0.02})₂(GaO₄)₃ of a stoichiometric composition. Then, dry mixing was performed using a mortar and a pestle, and a raw material for calcination was obtained.

The above-described calcination raw material was moved to an alumina crucible having a lid and was calcined for 2 hours in air at 1600 °C using a box-type electric furnace, and then the calcined material was lightly cracked to obtain a phosphor of an example 1. Note that the sample after calcination was confirmed to be Y₃(Ga_{0.98}, Cr_{0.02})₂(GaO₄)₃ using an X-ray diffraction method.

### (Evaluation of emission spectrum)

The emission spectrum of the phosphor was evaluated using a spectrofluorometer FP-6500 (manufactured by JASCO Corporation).

### [Example 2]

### (Preparation of phosphor)

An oxide phosphor was synthesized through a preparation procedure using a solid-state reaction. Specifically, an oxide phosphor expressed by the composition formula of Gd₃(Ga_{0.98}, Cr_{0.02})₂(GaO₄)₃ was synthesized. Note that the following compound powders were used as main raw materials in synthesizing the oxide phosphor.

Gadolinium oxide (Gd₂O₃): purity 3N, manufactured by Kojundo Chemical Lab. Co., Ltd.

Gallium oxide (Ga₂O₃): purity 4N, manufactured by Nippon Rare Metal, Inc.

Chromium oxide (Cr₂O₃): purity 3N, manufactured by Kojundo Chemical Lab. Co., Ltd.

First, the above raw materials were weighed to give a compound Gd₃(Ga_{0.98}, Cr_{0.02})₂(GaO₄)₃ of a stoichiometric composition. Then, dry mixing was performed using a mortar and a pestle, and a raw material for calcination was obtained.

The above-described calcination raw material was moved to an alumina crucible having a lid and was calcined for 2 hours in air at 1600 °C using a box-type electric furnace, and then the calcined material was lightly cracked to obtain a phosphor of an example 2. Note that the sample after calcination was confirmed to be Gd₃(Ga_{0.98}, Cr_{0.02})₂(GaO₄)₃ using an X-ray diffraction method.

### (Evaluation of emission spectrum)

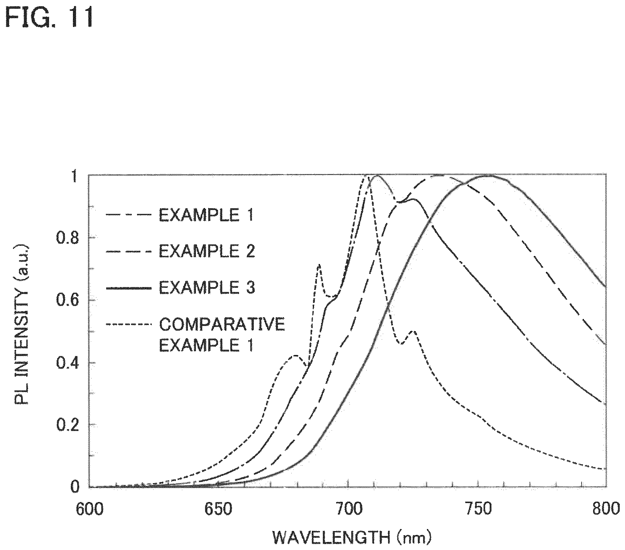

The emission spectrum of the phosphor was evaluated in the same manner as in the example 1. The result is illustrated in Fig. 11 and Table 1.

### [Example 3]

### (Preparation of phosphor)

An oxide phosphor was synthesized through a preparation procedure using a solid-state reaction. Specifically, an oxide phosphor expressed by the composition formula of (Gd_{0.75}, La_{0.25})₃(Ga_{0.98}, Cr_{0.02})₂(GaO₄)₃ was synthesized. Note that the following compound powders were used as main raw materials in synthesizing the oxide phosphor.

Gadolinium oxide (Gd₂O₃): purity 3N, manufactured by Kojundo Chemical Lab. Co., Ltd.

Lanthanum oxide (La₂O₃): purity 3N, manufactured by Kojundo Chemical Lab. Co., Ltd.

Gallium oxide (Ga₂O₃): purity 4N, manufactured by Nippon Rare Metal, Inc.

Chromium oxide (Cr₂O₃): purity 3N, manufactured by Kojundo Chemical Lab. Co., Ltd.

First, the above raw materials were weighed to give a compound (Gd_{0.75}, La_{0.25})₃(Ga_{0.98}, Cr_{0.02})₂(GaO₄)₃ of a stoichiometric composition. Then, dry mixing was performed using a mortar and a pestle, and a raw material for calcination was obtained.

The above-described calcination raw material was moved to an alumina crucible having a lid and was calcined for 2 hours in air at 1400 °C using a box-type electric furnace, and then the calcined material was lightly cracked to obtain a phosphor of an example 3. Note that the sample after calcination was confirmed to be (Gd_{0.75}, La_{0.25})₃(Ga_{0.98}, Cr_{0.02})₂(GaO₄)₃ using an X-ray diffraction method.

### (Evaluation of emission spectrum)

The emission spectrum of the phosphor was evaluated in the same manner as in the example 1. The result is illustrated in Fig. 11 and Table 1.

### [Comparative example 1]

### (Preparation of phosphor)

An oxide phosphor was synthesized through a preparation procedure using a solid-state reaction. Specifically, an oxide phosphor expressed by the composition formula of Y₃(Al_{0.98}, Cr_{0.02})₂(AlO₄)₃ was synthesized. Note that the following compound powders were used as main raw materials in synthesizing the oxide phosphor.

Yttrium oxide (Y₂O₃): purity 3N, manufactured by Shin-Etsu Chemical Co., Ltd.

Aluminum oxide (Al₂O₃): purity 3N, manufactured by Sumitomo Chemical Co., Ltd.

Chromium oxide (Cr₂O₃): purity 3N, manufactured by Kojundo Chemical Lab. Co., Ltd.

First, the above raw materials were weighed to give a compound Y₃(Al_{0.98}, Cr_{0.02})₂(AlO₄)₃ of a stoichiometric composition. Then, dry mixing was performed using a mortar and a pestle, and a raw material for calcination was obtained.

The above-described calcination raw material was moved to an alumina crucible having a lid and was calcined for 2 hours in air at 1600 °C using a box-type electric furnace, and then the calcined material was lightly cracked to obtain a phosphor of a comparative example 1. Note that the sample after calcination was confirmed to be Y₃(Al_{0.98}, Cr_{0.02})₂(AlO₄)₃ using an X-ray diffraction method.

### (Evaluation of emission spectrum)

The emission spectrum of the phosphor was evaluated in the same manner as in the example 1. The result is illustrated in Fig. 11 and Table 1.

Fig. 11 illustrates the emission spectrum excited at an excitation wavelength of 450 nm. Note that Fig. 11 also illustrates emission spectra of the example 2, the example 3, and the comparative example 1. Table 1 illustrates an emission peak wavelength λ_{MAX}, which is a peak wavelength of a fluorescence intensity maximum value peak indicating the fluorescence intensity maximum value in each emission spectrum. Table 1 also illustrates a spectral width (80% spectral width) W_{80%} at an intensity of 80% of the emission peak intensity (fluorescence intensity maximum value) of the fluorescence intensity maximum value peak. Moreover, Table 1 illustrates a 780-nm fluorescence intensity percentage L₇₈₀ₙₘ, which is the percentage of an emission intensity at the wavelength of 780 nm with respect to an emission peak intensity (fluorescence intensity maximum value) at the fluorescence intensity maximum value peak of the emission spectrum.

**[Table 1]**

| | Example 1 | Example 2 | Example 3 | Comparative example 1 |
|---|---|---|---|---|
| λ_{MAX} (nm) | 712 | 735 | 755 | 708 |
| W_{80%} (nm) | 33 | 54 | 62 | 11 |
| L₇₈₀ₙₘ (%) | 39 | 65 | 84 | 9 |

### (Summary of evaluation of emission spectra)

It was found that the phosphors of the examples 1 to 3 emitted wavelength-converted light containing a greater amount of a broad spectral component having a fluorescence intensity maximum value in a wavelength range exceeding 710 nm, than a linear spectral component having a fluorescence intensity maximum value in the wavelength range of 680 to 710 nm.

Note that the above-described linear spectral component is a long-afterglow light component based on the electron energy transition (spin-forbidden transition) of ²T₁ and ²E → ⁴A₂(t₂³) of Cr³⁺. The above-described broad spectral component is a short-afterglow light component based on an electron energy transition (spin-allowed transition) of ⁴T₂ → ⁴A₂.

Thus, with a light emitting device using the phosphors of the examples 1 to 3 as the first phosphor, it was found that a point light source containing a large amount of the near-infrared component could be easily made.

In a light emitting device using the phosphors of the examples 1 to 3 as the first phosphor, it was found that the saturation of the fluorescence output was low when excitation light of high light density was emitted, and achieving high output is easy.

In a light emitting device using the phosphors of the examples 1 to 3 as the first phosphor, it was found that the first wavelength-converted light 7 contains a large amount of a fluorescent component in the near-infrared wavelength range (650 to 1000 nm) in which light easily penetrates a living body, which is called a "biological window". Thus, it was found that a light irradiation type cosmetic apparatus using the phosphors of the examples 1 to 3 as the first phosphor increased the intensity of near-infrared light penetrating a living body, and enhanced the effect of suppressing unwanted hair growth, the performance in removing spots and freckles, and the like.

### [Comparative example 2]

### (Preparation of phosphor)

A nitride phosphor was synthesized through a preparation procedure using a solid-state reaction. Specifically, a nitride phosphor expressed by the composition formula of (Ca_{0.997}, Eu_{0.003})AlSiN₃ was synthesized. Note that the following compound powders were used as main raw materials in synthesizing the nitride phosphor.
Calcium nitride (Ca₃N₂): purity 2N, manufactured by TAIHEIYO CEMENT CORPORATION
Aluminum nitride (AlN): purity 3N, manufactured by Kojundo Chemical Lab. Co., Ltd.
Silicon nitride (Si₃N₄): purity 3N, manufactured by Denka Company Limited
Europium nitride (EuN): purity 2N, manufactured by TAIHEIYO CEMENT CORPORATION

First, the above raw materials were weighed in a glove box in an N₂ atmosphere to give a compound (Ca_{0.997}, Eu_{0.003})AlSiN₃ of a stoichiometric composition. Then, dry mixing was performed using a mortar and a pestle, and a raw material for calcination was obtained.

The above-described calcined raw material was moved to a boron nitride (BN) crucible having a lid and was calcined for 2 hours in an N₂(0.6MPa) pressurized atmosphere at 1600 °C using a pressurized atmosphere controlled electric furnace, and then the calcined material was lightly cracked to obtain a phosphor of a comparative example 2. Note that the sample after calcination was confirmed to be (Ca_{0.997}, Eu_{0.003})AlSiN₃ using an X-ray diffraction method.

### (Evaluation of emission lifetime)

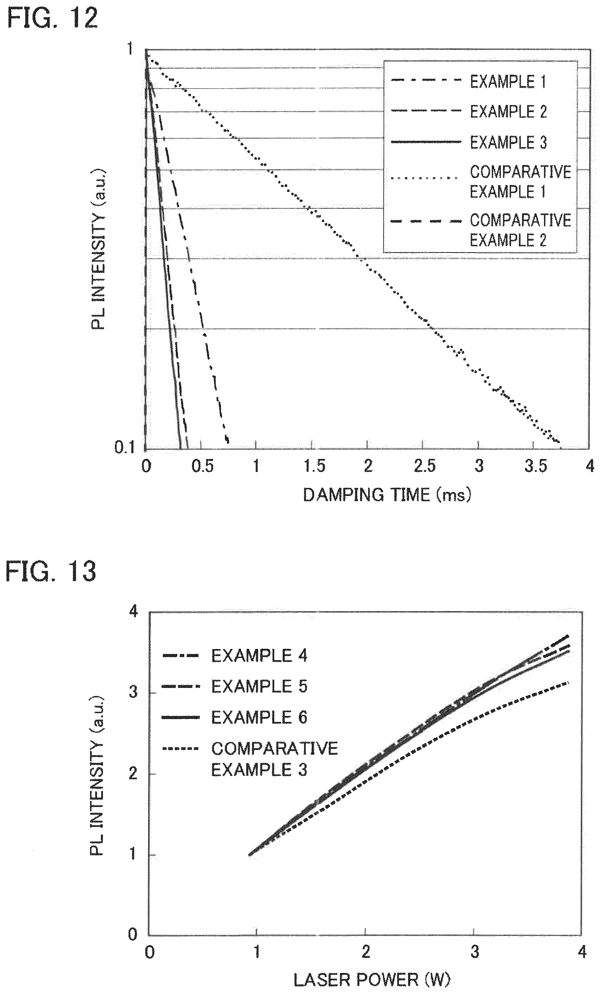

The emission lifetime of the phosphors was evaluated using a Quantaurus-Tau compact fluorescence lifetime measurement device (manufactured by Hamamatsu Photonics K.K.). The result is illustrated in Fig. 12 and Table 2.

Fig. 12 illustrates the emission lifetime of the example 1. Note that Fig. 12 also illustrates the emission lifetimes of the example 2, the example 3, the comparative example 1, and the comparative example 2.

Table 2 illustrates the time to reach 1/10 of the maximum emission intensity (1/10 afterglow): τ_{1/10}.

**[Table 2]**

| | Example 1 | Example 2 | Example 3 | Comparative example 1 | Comparative example 2 |
|---|---|---|---|---|---|
| τ_{1/10} (ms) | 0.78 | 0.38 | 0.32 | 3.81 | 0.0017 |

### (Summary of evaluation of emission lifetime)

It was found that the phosphors of the examples 1 to 3 emitted wavelength-converted light containing a greater amount of a short-afterglow near-infrared component present in a wavelength range exceeding 710 nm, than a long-afterglow linear spectral component having a fluorescence intensity maximum value within the wavelength range of 680 to 710 nm.

Note that the above-described long-afterglow linear spectral component is the light component based on the ²T₁ and ²E → ⁴A₂ electron energy transition (spin-forbidden transitions) of Cr³⁺. The above-described short-afterglow near-infrared component is a light component based on the electron energy transition (spin-allowed transition) of ⁴T₂ → ⁴A₂.

In a light irradiation type cosmetic apparatus using the above-described phosphors of the examples 1 to 3 as the first phosphor, it was found that a large amount of the near-infrared component is obtained, the saturation of the fluorescence output was low when high-output density light emitted from a solid-state light source having a rated output of 1 W or more was emitted, and achieving high output is easy.

### [Example 4]

### (Preparation of sintered body)

An amount of 1.0 g of phosphor powder of the example 1 was molded using a hydraulic press machine under a pressure of 210 MPa to produce a compact having a diameter of 13 mm. A sintered compact of an example 4 was obtained by calcining the compact for 1 hour in air at 1400 °C using a box-type electric furnace.

### [Example 5]

### (Preparation of sintered body)

An amount of 1.0 g of phosphor powder of the example 2 was molded using a hydraulic press machine under a pressure of 210 MPa to produce a compact having a diameter of 13 mm. A sintered compact of an example 5 was obtained by calcining the compact for 1 hour in air at 1400 °C using a box-type electric furnace.

### [Example 6]

### (Preparation of sintered body)

An amount of 1.0 g of phosphor powder of the example 3 was molded using a hydraulic press machine under a pressure of 210 MPa to produce a compact having a diameter of 13 mm. A sintered compact of an example 6 was obtained by calcining the compact for 1 hour in air at 1400 °C using a box-type electric furnace.

### [Comparative example 3]

### (Preparation of sintered body)

An amount of 0.5 g of phosphor powder of the comparative example 2 was molded using a hydraulic press machine under a pressure of 210 MPa to produce a compact having a diameter of 13 mm. A sintered compact of a comparative example 3 was obtained by calcining the compact for 2 hours in an N₂(0.6MPa) pressurized atmosphere at 1700 °C using a pressurized atmosphere controlled electric furnace.

### (Evaluation of fluorescence output saturation)

For a fluorescence output saturation property, a phosphor was irradiated with blue LD light having a peak wavelength of 450 nm using an integrating sphere, and the emission of light by a phosphor pellet was observed using a multichannel spectrometer. Here, the rated output of blue LD light was varied from 0.93 to 3.87 W. The irradiation area of the phosphor was 0.785 mm².

Fig. 13 illustrates the fluorescence output saturation property of the examples 4 to 6 and the comparative example 3. The lifetime of the Cr³⁺-activated phosphor was found to be extremely long compared to that of the Eu²⁺-activated phosphor. It was found that the Cr³⁺-activated phosphor is capable of maintaining a high emission efficiency even in a range where the power density of the excitation light is high, despite its long emission lifetime.

The invention is defined in the appended claims.

### INDUSTRIAL APPLICABILITY

The present disclosure is capable of providing a light irradiation type cosmetic apparatus that emits high-output light having a high proportion of a near-infrared fluorescent component under excitation with high-output light, and an electronic device using the light irradiation type cosmetic apparatus.

### REFERENCE SIGNS LIST

- 1, 1A, 1B, 1C, 1D, 1E: Light emitting device
- 2: Light source
- 3, 3A, 3B, 3C, 3D, 3E: Wavelength converter
- 4: First phosphor
- 6: Primary light
- 7: First wavelength-converted light
- 8: Second phosphor
- 9: Second wavelength-converted light
- 10: Light irradiation type cosmetic apparatus
- 15: Output light
- 20: Body part
- 21: Housing
- 23: Head-side end part
- 30: Head part
- 31: Head part body
- 41: Power button
- 50: Driving power source
- 110: Irradiation port
- 200: Solid-state light emitting element

## Claims

1. A light irradiation type cosmetic apparatus (10), comprising:
a light emitting device (1) including: a light source (2) configured to emit primary light (6); and a wavelength converter (3) including a first phosphor (4) configured to absorb the primary light (6) and convert the primary light (6) into first wavelength-converted light (7) having a wavelength longer than that of the primary light (6), wherein
the light source (2) is a solid-state light source having a rated output of 1 W or more,
the primary light (6) is light emitted from the solid-state light source,
the first wavelength-converted light (7) contains fluorescence based on an electron energy transition of Cr³⁺,
the first wavelength-converted light (7) has a fluorescence intensity maximum value in a wavelength range exceeding 710 nm,
wherein the light source (2) includes multiple solid-state light emitting elements (200),
wherein the light source (2) and the wavelength converter (3) are arranged in such a manner that the primary light (6) emitted from the multiple solid-state light emitting elements (200) of the light source (2) is emitted on the wavelength converter (3),
the light irradiation type cosmetic apparatus (10) includes a body part (20) and a head part (30) comprising an irradiation port (110), and
the light irradiation type cosmetic apparatus (10) is capable of irradiating human skin with an output light (15), including the primary light (6) and the first wavelength-converted light (7), generated at the body part (20), through the irradiation port (110) provided in the head part (30).

2. The light irradiation type cosmetic apparatus (10) according to claim 1, wherein
the primary light (6) has a power density of 0.5 W/mm² or more.

3. The light irradiation type cosmetic apparatus (10) according to claim 1 or 2, wherein
the first phosphor (4) has a garnet crystal structure.

4. The light irradiation type cosmetic apparatus (10) according to any one of claims 1 to 3, wherein
the light emitting device (1) further includes a second phosphor (8) configured to convert to second wavelength-converted light (9) that is different from the first wavelength-converted light (7).

5. The light irradiation type cosmetic apparatus (10) according to any one of claims 1 to 4, wherein
the first phosphor (4) contains two or more Cr³⁺-activated phosphors.

6. The light irradiation type cosmetic apparatus (10) according to any one of claims 1 to 5, wherein
the primary light (6) has an intensity maximum value within a wavelength range of 600 nm or more and less than 710 nm.

7. The light irradiation type cosmetic apparatus (10) according to any one of claims 1 to 5, wherein
the first wavelength-converted light (7) has a fluorescence intensity maximum value in a wavelength range of 710 nm or more and less than 900 nm.

8. An electronic device, comprising:
the light irradiation type cosmetic apparatus (10) according to any one of claims 1 to 7.

## Patentansprüche

1. Kosmetischer Apparat des Lichtbestrahlungstyps, umfassend
eine Licht emittierende Vorrichtung (1), enthaltend: eine Lichtquelle (2), konfiguriert, um Primärlicht (6) zu emittieren; und einen Wellenlängenkonverter (3), enthaltend einen ersten Leuchtstoff (4), konfiguriert, um das Primärlicht (6) zu absorbieren und das Primärlicht (6) in ein erstes wellenlängenkonvertiertes Licht (7) mit einer Wellenlänge, länger als die des Primärlichts (6), zu konvertieren, wobei
die Lichtquelle (2) eine Festkörperlichtquelle mit einer Nennleistung von 1 W oder mehr ist,
das Primärlicht (6) von der Festkörperlichtquelle emittiertes Licht ist,
das erste wellenlängenkonvertierte Licht (7) Fluoreszenz auf Grundlage eines Elektronenenergieübergangs von Cr³⁺ enthält,
das erste wellenlängenkonvertierte Licht (7) einen Fluoreszenzintensitätsmaximalwert in einem 710 nm überschreitenden Wellenlängenbereich aufweist,
wobei die Lichtquelle (2) mehrere Licht emittierende Festkörperelemente (200) enthält,
wobei die Lichtquelle (2) und der Wellenlängenkonverter (3) so angeordnet sind, dass das von den mehreren Licht emittierenden Festkörperelementen (200) der Lichtquelle (2) emittierte Primärlicht (6) auf den Wellenlängenkonverter (3) emittiert wird,
wobei der kosmetische Apparat des Lichtbestrahlungstyps (10) ein Körperteil (20) und ein Kopfteil (30), umfassend einen Bestrahlungsanschluss (10), enthält, und
wobei der kosmetische Apparat des Lichtbestrahlungstyps (10) in der Lage ist, menschliche Haut mit einem Ausgangslicht (15), umfassend das Primärlicht (6) und das erste wellenlängenkonvertierte Licht (7), die am Körperteil (20) erzeugt werden, mittels des in dem Kopfteil (30) bereitgestellten Bestrahlungsanschluss (110) zu bestrahlen.

2. Kosmetischer Apparat des Lichtbestrahlungstyps (10) nach Anspruch 1, wobei das Primärlicht (6) eine Leistungsdichte von 0,5 W/mm² oder mehr aufweist.

3. Kosmetischer Apparat des Lichtbestrahlungstyps (10) nach Anspruch 1 oder 2, wobei der erste Leuchtstoff (4) eine Granatkristallstruktur aufweist.

4. Kosmetischer Apparat des Lichtbestrahlungstyps (10) nach einem der Ansprüche 1 bis 3, wobei die Licht emittierende Vorrichtung (1) weiter einen zweiten Leuchtstoff (8) enthält, der konfiguriert ist, um zu einem zweiten wellenlängenkonvertierten Licht (9) zu konvertieren, das vom ersten wellenlängenkonvertierten Licht (7) verschieden ist.

5. Kosmetischer Apparat des Lichtbestrahlungstyps (10) nach einem der Ansprüche 1 bis 4, wobei der erste Leuchtstoff (4) zwei oder mehr Cr³⁺-aktivierte Leuchtstoffe enthält.

6. Kosmetischer Apparat des Lichtbestrahlungstyps nach einem der Ansprüche 1 bis 5, wobei das Primärlicht (6) einen Intensitätsmaximalwert innerhalb eines Wellenlängenbereichs von 600 nm oder mehr und weniger als 710 nm aufweist.

7. Kosmetischer Apparat des Lichtbestrahlungstyps nach einem der Ansprüche 1 bis 5, wobei das erste wellenlängenkonvertierte Licht (7) einen Fluoreszenzintensitätsmaximalwert in einem Wellenlängenbereich von 710 nm oder mehr und weniger als 900 nm aufweist.

8. Elektronische Vorrichtung, umfassend:
den kosmetischen Apparat des Lichtbestrahlungstyps nach einem der Ansprüche 1 bis 7.

## Revendications

1. Appareil cosmétique de type à irradiation lumineuse (10), comprenant :
un dispositif d'émission lumineuse (1) incluant : une source lumineuse (2) configurée pour émettre de la lumière primaire (6) ; et un convertisseur de longueur d'onde (3) incluant un premier phosphore (4) configuré pour absorber la lumière primaire (6) et convertir la lumière primaire (6) en première lumière à longueur d'onde convertie (7) ayant une longueur d'onde plus longue que celle de la lumière primaire (6), dans lequel
la source lumineuse (2) est une source lumineuse à état solide ayant une sortie nominale de 1 W ou plus,
la lumière primaire (6) est de la lumière émise depuis la source lumineuse à état solide,
la première lumière à longueur d'onde convertie (7) contient de la fluorescence basée sur une transition d'énergie électronique de Cr³⁺,
la première lumière à longueur d'onde convertie (7) a une valeur maximale d'intensité de fluorescence dans une plage de longueur d'onde dépassant 710 nm,
dans lequel la source lumineuse (2) inclut de multiples éléments d'émission lumineuse à état solide (200),
dans lequel la source lumineuse (2) et le convertisseur de longueur d'onde (3) sont agencés de telle sorte que la lumière primaire (6) émise depuis les multiples éléments d'émission lumineuse à état solide (200) de la source lumineuse (2) est émise sur le convertisseur de longueur d'onde (3),
l'appareil cosmétique de type à irradiation lumineuse (10) inclut une partie de corps (20) et une partie de tête (30) comprenant un port d'irradiation (10), et
l'appareil cosmétique de type à irradiation lumineuse (10) est capable d'irradier de la peau humaine avec une lumière sortie (15), incluant la lumière primaire (6) et la première lumière à longueur d'onde convertie (7), générée au niveau de la partie de corps (20), à travers le port d'irradiation (110) prévu dans la partie de tête (30).

2. Appareil cosmétique de type à irradiation lumineuse (10) selon la revendication 1, dans lequel
la lumière primaire (6) a une densité de puissance de 0,5 W/mm² ou plus.

3. Appareil cosmétique de type à irradiation lumineuse (10) selon la revendication 1 ou 2, dans lequel
le premier phosphore (4) a une structure cristalline de grenat.

4. Appareil cosmétique de type à irradiation lumineuse (10) selon l'une quelconque des revendications 1 à 3, dans lequel
le dispositif d'émission lumineuse (1) inclut en outre un second phosphore (8) configuré pour convertir en seconde lumière à longueur d'onde convertie (9) qui est différente de la première lumière à longueur d'onde convertie (7).

5. Appareil cosmétique de type à irradiation lumineuse (10) selon l'une quelconque des revendications 1 à 4, dans lequel
le premier phosphore (4) contient deux phosphores à Cr³⁺ activé ou plus.

6. Appareil cosmétique de type à irradiation lumineuse (10) selon l'une quelconque des revendications 1 à 5, dans lequel
la lumière primaire (6) a une valeur maximale d'intensité au sein d'une plage de longueur d'onde de 600 nm ou plus et moins de 710 nm.

7. Appareil cosmétique de type à irradiation lumineuse (10) selon l'une quelconque des revendications 1 à 5, dans lequel
la première lumière à longueur d'onde convertie (7) a une valeur maximale d'intensité de fluorescence dans une plage de longueur d'onde de 710 nm ou plus et moins de 900 nm.

8. Dispositif électronique, comprenant :
l'appareil cosmétique de type à irradiation lumineuse (10) selon l'une quelconque des revendications 1 à 7.
